# EUROPEAN PATENT APPLICATION

(11) **EP 3 527 673 A1**
(43) Date of publication of application: **21.08.2019**
(21) Application number: 18157046.6
(22) Date of filing: 15.02.2018
(51) Int. Cl.: C12Q 1/6823

(54) **SEQUENCING METHOD**

(71) Applicant: QIAGEN GmbH, 40724 Hilden (DE)
(72) Inventor: FOCKE, Maximilian, 40724 Hilden (DE); KÖBER, Sebastian, 40724 Hilden (DE)
(74) Representative: Roth, Carla

(57) **Abstract**

The present invention provides a method for determining the sequence of a nucleic acid template comprising
a) contacting the nucleic acid template with a hybridizing sequencing reagent comprising a detectable label under suitable conditions to provide a hybrid comprising a detectable label;
b) releasing the detectable label from the hybrid; and
c) separating released detectable label from the nucleic acid template;
wherein the method further comprises d) detecting separated detectable label for determining the sequence. Decoupling the sequencing reaction from the detection step allows to improve various sequencing methods. Also provided are corresponding sequencing systems.

## Description

The present invention *inter alia* relates to methods and systems for nucleic acid sequencing which use a hybridizing sequencing reagent comprising a detectable label, such as e.g. fluorescently labelled nucleotides or fluorescently labelled probes, to determine the sequence of a nucleic acid template.

### BACKGROUND OF THE INVENTION

Nucleic acid sequencing is the process of determining the precise order of nucleotides within a nucleic acid molecule. In DNA sequencing, it includes the step of determining the order of the four nucleobases-adenine, guanine, cytosine, and thymine-in a strand of DNA. Traditional sequencing methods (e.g. Sanger sequencing) are being replaced by next-generation sequencing technologies. Next-generation sequencing (NGS) allows to determine the sequence of DNA and RNA (which usually is reverse transcribed first to cDNA) molecules in a massively parallel manner. Next-generation sequencing is widely used to sequence e.g. exomes, genomes, circulating nucleic acids, targeted panels or mitochondrial DNA.

Prior art next-generation sequencing approaches are reviewed e.g. in Goodwin et al., Nature Reviews, June 2016, Vol. 17: pp. 333 - 351 "Coming of age: ten years of next-generation sequencing technologies", Yohe et al., Arch Pathol Lab Med, November 2017, Vol. 141: pp. 1544 - 1557 "Review of Clinical Next-Generation Sequencing" and Masoudi-Nejad, Chapter 2 "Emergence of Next-Generation Sequencing in "Next Generation Sequencing and Sequence Assembly" SpringerBriefs in Systems Biology, 2013, all herein incorporated by reference. Current next-generation sequencing approaches share common sample preparation steps to provide the nucleic acid template that is then sequenced. These common steps usually include steps such as nucleic acid extraction, library preparation, target enrichment and clonal amplification. The so prepared nucleic acid template is then ready to be sequenced. Widely used sequencing approaches fall under two broad categories: sequencing by ligation (SBL) and sequencing by synthesis (SBS). In most SBL and SBS approaches, DNA is clonally amplified on a solid surface. Having multiple identical copies of a DNA fragment in a defined area ensures that the signal used to determine the sequence can be distinguished from background noise. Both approaches commonly use hybridizing sequencing reagents comprising a detectable label to determine the sequence.

In sequencing by ligation (SBL) approaches, the hybridizing sequencing reagent comprising a detectable label is a probe sequence that is bound to a detectable label, usually a fluorophore. It hybridizes to a DNA fragment and is ligated to an adjacent oligonucleotide for imaging. SBL approaches usually involve the hybridization and ligation of labelled probe and anchor sequences to a DNA strand. The probes encode one or two known bases (one-base-encoded probes or two-base-encoded probes) and a series of degenerate or universal bases, driving complementary binding between the probe and template, whereas the anchor fragment encodes a known sequence that is complementary to an adapter sequence and provides a site to initiate ligation. After ligation, the template is imaged and the known base or bases in the probe are identified. The emission spectrum of the fluorophore indicates the identity of the base or bases complementary to specific positions within the probe. A new cycle begins after complete removal of the anchor-probe complex or through cleavage to remove the fluorophore and to regenerate the ligation site. Commercially available platforms that are based on sequencing by ligation are SOLiD sequencing and Complete Genomics (see Goodwin et al, 2016).

Sequencing by synthesis (SBS) is a term used to describe numerous DNA-polymerase-dependent methods. In sequencing by synthesis (SBS) approaches, a polymerase is used and a signal, such as a fluorophore or a change in ionic concentration, identifies the incorporation of a nucleotide into an elongating strand. Commonly used SBS approaches can be classified e.g. as cyclic reversible termination (CRT) or as single-nucleotide addition (SNA). CRT approaches are widely used in the art and use a hybridizing sequencing reagent comprising a detectable label. Usually, a nucleotide analogue comprising a detectable label, such as a fluorescent label, is used. Different types of nucleotide analogous comprising a detectable label can be used for CRT based methods. Labelled nucleotide analogues commonly used in CRT based methods are also referred to sometimes as reversible terminators or terminator molecules. Different embodiments are used, such as e.g. 3'-O-blocked reversible terminators and 3'-unblocked reversible terminators (see e.g. Chen et al, 2013 "The History and Advances of Reversible Terminators Used in New Generations of Sequencing Technology" Genomics Proteomics Bioinformatics 11 (2013) 34-40 and Chen et al, 2014, "DNA polymerases drive DNA sequencing-by synthesis technologies: both past and present", Frontiers in Microbiology, Volume 5, Article 305, p. 1 to 11; herein incorporated by reference). The 3'O-blocked reversible terminators are similar to those used in Sanger sequencing, in which the ribose 3'-OH group of the nucleotide is blocked by a terminating group, also referred to as capping moiety, thus preventing elongation after the incorporation of a labelled nucleotide analogue. However, the 3'O-blocked reversible terminators used in next generation sequencing methods comprise a reversible terminating group which accordingly can be removed and the label, e.g. a fluorescent dye, is also attached removable, e.g. via a cleavable linker. The main steps of commonly used sequencing approaches are summarized subsequently. To begin the sequencing process, an immobilized DNA template is usually primed by a sequence that is complementary to an adapter region (or a self-priming DNA template is used), which will initiate polymerase binding to this double-stranded DNA (dsDNA) region. During each sequencing cycle, a mixture of labelled nucleotide analogues, e.g. four individually labelled and 3'-blocked deoxynucleotides (dNTPs), is added. The incorporation of a single labelled nucleotide analogue to each elongating complementary strand provides a hybrid which comprises the detectable label of the nucleotide analogue that was incorporated. Unbound labelled nucleotide analogues are removed and the immobilized DNA template is imaged to identify based on the detectable label which nucleotide was incorporated into the hybrid thereby allowing to identify the complementary base of the nucleic acid template. The detectable label (e.g. fluorophore) and the capping moiety are then removed and a new sequencing cycle can begin to identify the next consecutive base. Commercially available platforms using sequencing by synthesis based on cyclic reversible termination (CRT) are MiSeq, HiSeq, NextSeq etc. (Illumina) and GeneReader (QIAGEN) (see also Goodwin et al, 2016). In the Illumina platforms, after solid-phase template enrichment, a mixture of primers, DNA polymerase and nucleotide analogues are added to the flow cell. Each nucleotide analogue is blocked by a 3'-O-azidomethyl group and is labelled with a base-specific, cleavable fluorophore. During each sequencing cycle, the nucleic acid templates in each cluster will incorporate just one nucleotide analogue as the blocked 3' group prevents additional incorporations. After base incorporation, unincorporated nucleotide analogues are washed away and the slide is imaged by total internal reflection fluorescence (TIRF) microscopy using either two or four laser channels. The colour (or the lack or mixing of colours in the two-channel system used by NextSeq) identifies which base was incorporated in each cluster. The fluorescent dye is then cleaved and the 3'-OH is regenerated with a reducing agent (e.g. tris(2-carboxyethyl)phosphine ((TCEP)). The sequencing cycle of nucleotide analogue addition, elongation and cleavage is then repeated to identify the next base. In the GeneReader platform, after bead-based template enrichment, a mixture of primers, DNA polymerase and modified nucleotides are added to the flow cell. Each nucleotide analogue is blocked by a 3'-O-allyl group and some of the bases are labelled with a base-specific, cleavable fluorophore. After base incorporation, unincorporated nucleotide analogues are washed away and the slide can be imaged with a fluorescence microscope using four laser channels or LEDs. The dye is then cleaved and the 3'-OH is regenerated with the reducing agent (e.g. mixture of palladium and P(PhSO₃Na)₃ (TPPTS)). Hence, common sequencing by synthesis based methods share important core features. Various nucleic acid templates are attached to a support, also referred to herein as substrate. Subsequently, a nucleotide labelled with a fluorescent dye is hybridized and attached to the growing oligonucleotide chain with a DNA polymerase. Fluorescent signals emitted can be detected and the type of nucleotide attached to the oligonucleotide can be identified. After detection, the fluorescent dye is cleaved off and the next synthesis cycle is carried out, in which a new labelled nucleotide is attached to the growing chain. By carrying out multiple cycles, the sequence of a growing oligonucleotide chain can be determined in a stepwise manner. As discussed, different labeling formats for nucleotide analogues are known in the prior art.

These prior art methods that use a hybridizing sequencing reagent comprising a detectable label to determine the sequence of a nucleic acid template have drawbacks. They consume a high amount of sequencing chemistry, which in turn leads to high costs. The high consumption is triggered by the current design of the liquid processing through pumps and tubes, which leads to high dead volumes. Furthermore, the current methods have high turn-around-times and pose a risk to damage the nucleic acid template.

The invention aims at avoiding drawbacks of the prior art methods. It is an object to provide an improved method for determining the sequence of a nucleic acid template. In particular, it is an object of the invention to provide a method for determining the sequence of a nucleic acid template which reduces turn-around-times and/or reduces the risk to damage the nucleic acid template. It is furthermore an object to reduce costs.

### SUMMARY OF THE INVENTION

The invention is defined by the appended claims.

According to a first aspect, a method for determining the sequence of a nucleic acid template is provided, comprising
a) contacting the nucleic acid template with a hybridizing sequencing reagent comprising a detectable label under suitable conditions to provide a hybrid comprising a detectable label;
b) releasing the detectable label from the hybrid; and
c) separating released detectable label from the nucleic acid template;
wherein the method further comprises d) detecting the separated detectable label for determining the sequence.

In contrast to prior art sequencing methods, the detectable label that is used to identify the sequence of the nucleic acid template is separated from the nucleic acid template prior to detection. Physically separating the detectable label from the nucleic acid template prior to detection allows to identify the detectable label independently of the presence of the nucleic acid template. This has several important advantages.

In the prior art methods, the sequential processing of the sequencing reaction and the detection of the detectable label that is used to identify the sequenced base(s) leads to long turn-around-times. By separating the detectable label from the nucleic acid template prior to detection, the method of the invention decouples the sequencing reaction from the detection of the detectable label that was incorporated during a sequencing cycle. This allows to accelerate the sequencing turn-around-time. Decoupling the sequencing reaction from the detection of the detectable label moreover significantly simplifies the detection and provides more flexibility with respect to the detection. It e.g. allows to reduce the number of detection steps required for determining the sequence of a given nucleic acid template compared to prior art methods. As discussed above, the prior art methods require the detection of the incorporated detectable label during each sequencing cycle. E.g. in prior art sequencing methods that use four differently labelled nucleotide analogues, the incorporation of the labelled nucleotide analogue must be detected during each sequencing cycle and occurs in the presence of the nucleic acid template, namely while it is bound to the nucleic acid template. E.g. when using hybridizing sequencing reagents comprising four different fluorescent dye (one for each base), detection requires making four images with the camera (one for each label) during each sequencing cycle in order to identify the incorporated label and thus to determine the incorporated nucleotide analogue. Thus, for sequencing a nucleic acid template having an exemplary length of 50 nucleotides and using a prior art system that requires 4 images to identify a base, overall 200 images are required in order to identify the sequence of the nucleic acid template. This is time consuming and leads to long turn-around-times. It moreover requires large data storage capacities if multiple different nucleic acid templates are sequenced in parallel. With the present invention, these drawbacks can be avoided. As is described herein, after incorporating the hybridizing sequencing reagent comprising a detectable label (e.g. a nucleotide analogue comprising a detectable label) under suitable conditions to provide a hybrid, the detectable label which is used to identify the base is released from the hybrid and furthermore separated from the nucleic acid template. This can be done e.g. by transferring detectable label released in a sequencing cycle in a liquid medium to a detection substrate. After the detectable labels released during the individual sequencing cycles were transferred to the detection substrate, the detection substrate can then be moved to a detection device to identify the detectable labels comprised in the spots that were provided on the detection substrate. This e.g. allows to identify all sequenced bases present on a detection substrate in a subsequent detection process. Thus, instead of taking 4 images after each sequencing cycle as is done in prior art methods that require 4 images to identify a base (see above), the 4 images can be taken decoupled from the sequencing reaction, thereby allowing to identify multiple bases in one detection step. Thus, significantly less images are required to identify the sequence of the nucleic acid template. The present method is thus less time consuming compared to prior art methods as the turn-around-time can be increased. Moreover, less storage capacity for storing the images is required.

Furthermore, as is described herein, decoupling the sequencing reaction from the detection of the detectable label(s) allows to perform further sequencing cycles in parallel to the detection. This is particularly advantageous if multiple different nucleic acid templates are sequenced and analyzed in parallel as it is desired for high throughput applications. After the detectable labels of the first sequencing cycle were separated from the nucleic acid templates, further processing steps (e.g. wash steps) and/or further sequencing cycles can be directly performed while the detection of the detectable labels of the first sequencing cycle may occur in parallel. This also allows to reduce the turn-around-time. In prior art methods, the next sequencing cycle can only begin after the detectable label incorporated in the hybrid has been detected.

Moreover, direct imaging of the nucleic acid template through fluorescence to identify the incorporated detectable label as it is done in prior art methods poses a potential damage risk for the nucleic acid template. It can lead to a decay of signal for extended cycles of sequencing. To reduce such damage, prior art methods protect the nucleic acid sample by suitable buffer systems during the imaging process in order to prevent light induced damage to the sample. Those buffers need to be removed from the system by suitable wash steps, before the next steps can be performed. With the present invention that decouples imaging and sequencing this damage risk is avoided. This in turn allows omitting the functional buffers for the protection of the nucleic acid template during the imaging process and the associated wash processes. Therefore, the method of the invention requires less individual steps than prior art methods and allows to reduce reagent consumption.

Furthermore, decoupling the sequencing reaction from the detection of the label of the incorporated hybridizing agent allows to provide more compact sequencing systems. A respective sequencing system that is designed to perform the method of the present invention does e.g. not mandatorily require bulky optical detection units. Instead, the detection unit can be provided separately and can be designed according to the desired resolution of the imaging process, which is directly proportional to the output density of the system. Furthermore, fluidic tubings and reservoirs can be reduced, e.g. when using a printing device as it is described herein.

The method of the invention therefore provides important advantages and makes a significant contribution to the art. It can be used in conjunction with established sequencing methods that are based on the use of a hybridizing sequencing reagent comprising a detectable label, such as 3'-O-blocked reversible terminators and 3'-unblocked reversible terminators comprising a detectable label or probes comprising a detectable label. Such methods are described in the background of the invention and also below and in particular are methods that rely on sequencing by synthesis (SBS) or sequencing by ligation (SBL).

According to a second aspect, a sequencing system for performing the sequencing method according to the first aspect is provided.

According to a third aspect, the present invention pertains to the use of a printing device for transferring a detectable label that was released from a nucleic acid hybrid generated in the course of a sequencing reaction to a bypass location, preferably a detection substrate, wherein the transferred detectable label is detected to identify the detectable label.

Other aspects, objects, features, and advantages of the present application will become apparent to those skilled in the art from the following description and appended claims. It should be understood, however, that the following description, appended claims, and specific examples, while indicating preferred embodiments of the application, are given by way of illustration only.

### DETAILED DESCRIPTION OF THE INVENTION

### METHOD FOR DETERMINING THE SEQUENCE OF A NUCLEIC ACID TEMPLATE

According to a first aspect, a method for determining the sequence of a nucleic acid template is provided, comprising
a) contacting the nucleic acid template with a hybridizing sequencing reagent comprising a detectable label under suitable conditions to provide a hybrid comprising a detectable label;
b) releasing the detectable label from the hybrid; and
c) separating released detectable label from the nucleic acid template;
wherein the method further comprises d) detecting the separated detectable label for determining the sequence.

Steps a) and b) can be performed as it is known from prior art sequencing methods. Such sequencing methods and suitable conditions for performing steps a) and b) are well-known to the skilled person and are also described in the background of the invention. In contrast to prior art methods, the present method separates in step c) the released detectable label from the nucleic acid template prior to detecting the separated detectable label for determining the sequence in step d). Important advantages of the method according to the first aspect are described in the summary of the invention. As discussed herein, this allows to decouple the sequencing reaction and optionally further processing steps from the detection of the incorporated label and hence the determination of the sequence. The detection and identification of the detectable label can be performed independently of further sequencing cycles and optionally further processing steps that involve the nucleic acid template. The method and advantageous embodiments thereof are described in further detail below.

According to one embodiment, separating in step c) involves transferring released detectable label to a bypass-location. A bypass-location in particular refers to a location that is different to a location where the nucleic acid template is processed for sequencing. After released detectable label was transferred to the bypass-location, the nucleic acid template can be directly further processed (e.g. washed) and subjected to further sequencing cycles. The detection of the detectable label can thereby be decoupled from the further processing of the nucleic acid template. Providing a bypass-location therefore allows to perform the detection of the released detectable label independently from further processing steps that involve the nucleic acid template. As discussed above, detection of the detectable label incorporated during a sequencing cycle can be time consuming. Likewise, further processing steps necessary to prepare the nucleic acid template for a (further) sequencing cycle can also be time consuming and can e.g. lead to intermediate fluid products having to be discharged, before a further sequencing cycle can be performed. Such intermediate steps can, for example, be the washing of the nucleic acid template after a sequencing cycle. The liquid produced in this washing step typically needs to be discharged and thus removed before a further sequencing cycle can be initiated. The method according to the present invention allows the steps of preparing the nucleic acid template for a further sequencing cycle (if needed) as well as the steps of a further sequencing cycle to be performed in parallel to the detection of the separated detectable label that was released in the first sequencing cycle. The released detectable label that was separated and transferred to a bypass-location can be further processed and detected independently of the nucleic acid template. The released detectable label in the bypass-location is physically separated from the nucleic acid template and thus the flow of fluids over the nucleic acid template in further reaction steps performed for preparing or performing further sequencing cycles.

### Transfer to a detection substrate

Preferably, separating in step c) involves transferring released detectable label comprised in a liquid medium to a detection substrate. The detection substrate is physically separated and thus spaced apart from the location where the nucleic acid template is located.

The transfer to a detection substrate is an embodiment of a transfer to a bypass location. During separation step c), the released detectable label comprised in a liquid can be transferred to a definite location on the detection substrate. E.g., the released detectable label comprised in a liquid can be provided in form of a spot on the detection substrate. The transferred spot comprising the detectable label can thereby identify e.g. one base of the nucleic acid template as the result of a sequencing cycle if using labelled nucleotide analogues as hybridizing sequencing reagent comprising a detectable label. It is also possible to transfer detectable label released in a single sequencing cycle in form of two or more spots on the detection substrate. Providing two or more spots of the detectable label released during a single sequencing cycle can increase the specificity. In prior art methods, the detectable label is determined while it is present in the formed hybrid and thus is associated with the nucleic acid template. Thus, in prior art methods during detection, only one image per cycle is obtained which leads to a single value for the confidence of a correct base-call. This confidence is typically found to be decreasing throughout the sequencing process, which leads to a limited length of overall read-length that can be considered data of suitable confidence. Printing the detectable label can accommodate the decay of signal at later stages of the sequencing process that can be leveraged to increase average read length of suitable confidence.

The detection substrate can be a solid substrate. The solid substrate can be a self-supporting substrate. A self-supporting substrate is considered to be a substrate that essentially keeps its shape, even if it is only held at one end. A substrate that essentially keeps its shape, even if it is only held at one end, is considered to be a substrate that in a situation, where it is supported at two opposite ends extends along a first line, and still extends along the same first line or extends along a second line that is angled by less than 45°, preferably by less than 20° to the first line, if the support at one end is taken away. In an alternative, the solid substrate can be a non-self-supporting substrate, for example a foil. A solid substrate that is a non-self-supporting substrate is made suitable to carry a drop of a liquid medium, by supporting the non-self-supporting substrate at least at two sides, preferably at least at two opposing ends of the substrate. Preferably, the non-self-supporting substrate is supported by being clamped by support clamps. Preferably, the non-self-supporting substrate is clamped in such a manner that the non-self-supporting substrate is tensioned or preferably stretched. The actual detection can be performed using the liquid medium comprising the released detectable label provided on the substrate or in a dried state after evaporation of the carrier fluid.

The detection substrate can be a transparent substrate, preferably a clear transparent substrate. An opaque transparent substrate may also be feasible.

The material of the detection substrate can be made e.g. of glass, silicon or a plastic material. Suitable substrate materials are known to the skilled person and include, but are not limited to borosilicate glass, fused silica, cyclic olefin-copolymers, polycarbonates and polymer-metal composites. As discussed above, the detection substrate receives an aliquot, e.g. one or more droplets, of a liquid medium comprising the detectable label. The substrate may comprise hydrophobic and hydrophilic regions. The hydrophilic regions may form discrete spots that are at least partially, preferably completely surrounded by hydrophobic regions. This supports the ordered deposition of the liquid medium in a definite location on the detection substrate, as the deposited liquid medium will become located in the hydrophilic region. The hydrophobic regions surrounding the hydrophilic spots prevent a cross-contamination between adjacent hydrophilic regions. This is advantageous if providing a detection substrate comprising numerous densely arranged hydrophilic spots. In one embodiment, an aliquot, e.g. one or more droplets, of the liquid medium comprising the detectable label is transferred to the detection substrate onto a hydrophilic area, e.g. a hydrophilic spot. According substrates are known in the art from various microarray formats. According to one embodiment, a slightly hydrophobic surface is provided, e.g. having a global contact angle of less than 90°C, less than 70°C or less than 50°C. The surface where the detectable label is placed is wettable.

The detection substrate may have any suitable shape and can be e.g. rectangular or circular. In a preferred embodiment, the detection substrate has a rectangular shape. It may have an essentially flat surface.

In one embodiment, the detection substrate is part of the detection device. The detection device may be an optical sensor such as a CMOS or CCD sensor. Other detection devices, such as imaging systems, are also well-known in the art. The detection may be carried out directly on the individual pixels of the sensor. For non-contact detection, a lens-less detection system may be utilized based on the resolution of the sensor, and the density of spots to be investigated. The detection device may further have a lens or a suitable lens-system of a given magnification. In embodiments, the lens can be used as detection substrate to which released detectable label is transferred. The detection device suitable to detect a characteristic property of the detectable label on the substrate can be an optical detection device, preferably a camera, a lens camera system, or a line-scan camera. Examples of optical devices include CCD cameras, confocal imaging, total internal reflection fluorescence, zero-mode waveguide and the like.

In one embodiment, detecting in step d) comprises moving the detection substrate with the transferred detectable label to a detection device to identify the separated detectable label. The detection device may be an optical detection device (see e.g. above). It may comprise a sensor. The sensor may scan the spot(s) present on the detection substrate to detect and identify the detectable label transferred as the result of a sequencing cycle. This allows to determine at least one residue of the nucleic acid template to which the hybridizing sequencing reagent comprising the detectable label bound in step a) of a sequencing cycle and accordingly allows to determine the sequence of the nucleic acid template.

### Sequencing principles and used reagents

The method according to the present invention can be used in conjunction with any sequencing method that is based on the use of a hybridizing sequencing reagent comprising a detectable label to identify the sequence and thus the consecutive bases of a nucleic acid template based on the detectable label. Such sequencing methods are known to the skilled person and are also described in the background of the invention (see e.g. Goodwin, 2016, Yohe et al., 2017, and Masoudi-Nejad, 2013). The sequencing principle applied may be selected from (i) sequencing by synthesis (SBS) and (ii) sequencing by ligation (SBL). Preferably, the sequencing method is based on sequencing by synthesis, more preferably sequencing by synthesis by cyclic reversible termination.

In one embodiment, the hybridizing sequencing reagent comprising a detectable label is a nucleotide analogue comprising a nucleobase and a detectable label. Such nucleotide analogues are commonly used in conjunction with sequencing methods that are based on sequencing by synthesis (SBS), in particular sequencing by synthesis by cyclic reversible termination. Such methods are discussed in background of the invention and elsewhere herein (see also e.g. Goodwin 2016, Chen, 2013 and Chen, 2014). The nucleic acid template may thus be contacted with four nucleotide analogues (one for each base of the nucleic acid template), each optionally comprising a different detectable label in addition to the nucleobase. As is well-known in the art and discussed herein, different labelling formats are available so that the use of 4 different detectable labels (one label for each base) is only one preferred option among other suitable options. Reaction conditions are used that are suitable to provide the hybrid. Thus, a polymerase and optionally further reagents are added. Details are well-known to the skilled person. The complementary nucleotide analogue capable of hybridizing to the corresponding base of the nucleic acid template is incorporated (single-base extension), thereby providing a hybrid comprising the corresponding detectable label that identifies the incorporated nucleotide analogue (see step a) of claim 1). Unincorporated nucleotide analogues are then removed, e.g. by performing one or more wash steps. The incorporated detectable label that identifies the base is then released from the hybrid, e.g. cleaved off (see step b) of claim 1). Released detectable label is then physically separated from the nucleic acid template (see step c) of claim 1) prior to detecting the separated detectable label in order to identify the base based on the detectable label (see step d) of claim 1). Once a sufficient amount of released detectable label has been separated, e.g. by transferring one or more small droplets comprising released detectable label to a detection device, any remaining liquid comprising released detectable label can be removed and discarded. When using sequencing by synthesis by cyclic reversible termination, each sequencing cycle usually extends the hybrid formed with the nucleic acid template by one base.

In a further embodiment, the hybridizing sequencing reagent comprising a detectable label is an oligonucleotide probe comprising a detectable label. Such oligonucleotide probes comprising a detectable label are commonly used in sequencing methods that are based on sequencing by ligation (SBL) in order to identify the sequence of the nucleic acid template. Such methods are known to the skilled person and are also described in the background of the invention to which it is referred (see e.g. Goodwin, 2016). One well-known example of a sequencing method that is often based on sequencing by ligation is DNA nanoball sequencing. The nucleic acid template may be contacted with a pool of four differently labelled oligonucleotide probes which comprise degenerated nucleotides and at least one known base and a ligase, such as a T4 DNA ligase. Unincorporated oligonucleotide probes are removed. These SBL based methods also use the detectable label that is incorporated into the hybrid in order to identify the sequence of the nucleic acid template. Depending on the embodiment used, at least one incorporated base may remain in the hybrid after releasing the detectable label. In another embodiment that is e.g. commonly used for DNA nanoball sequencing, the entire labelled probe that was incorporated in step a) is removed in step b) thereby also releasing the detectable label from the hybrid while at the same time restoring the nucleic acid template (see e.g. Goodwin, 2016). These well-known methods that are based on sequencing by ligation can be significantly improved by the present invention which teaches to separate released detectable label from the nucleic acid template prior to detecting the separated detectable label in step d). Thus in contrast to prior art methods, the detection of the detectable label that was incorporated in step a) does not occur while the detectable label is present in the hybrid and thus while it is associated with the nucleic acid template. Instead, prior to detection, the detectable label is released from the hybrid and a sufficient amount of released detectable label is moreover separated from the nucleic acid template for detection. Once a sufficient amount of released detectable label has been separated, e.g. by transferring one or more small droplets comprising released detectable label (e.g. in form of a cleaved-off label or associated to the probe) to a detection device, any remaining liquid comprising released detectable label can be removed and discarded. However, as is described herein, DNA nanoball sequencing can also be performed using SBS based methods.

The hybridizing sequencing reagent comprises a detectable label, which means at least one detectable label. According to one embodiment, the hybridizing sequencing reagent comprises only one detectable label. Suitable detectable labels are well-known in the art. Common labels described in the art include fluorescent, luminescent, light-scattering and colorimetric labels. Detectable labels can rely on optical properties of the detectant, such as absorption and/or refraction, or may be of a non-optical transducer origin, such as magnetic labels. According to a preferred embodiment, the label is a fluorescent label, preferably a fluorescent dye. Suitable labels and in particular fluorescent labels such as fluorescent dyes are well known in the art and are also described in the documents referred to herein, see also US2014/0242579. As is also described therein, a hybridizing sequencing reagent, such as e.g. a reversible terminator, may comprise two labels, such as e.g. two dyes, wherein the two labels or dyes may optionally quench each other. Label or dye pairs that quench each other when they are in close proximity, e.g. FRET pairs, are also well-known in the art.

According to one embodiment, the hybridizing sequencing reagent comprises a cleavable linker to which the detectable label is attached. The detectable label can thus be attached via a cleavable linker in a nucleotide analogue as preferred embodiment of a hybridizing sequencing reagent that is commonly used in SBS sequencing methods. In an alternative embodiment, the detectable label is attached to an oligonucleotide probe as embodiment of a hybridizing sequencing reagent that is used in SBL sequencing methods. The cleavable linker may be a photocleavable linker or a chemically cleavable linker. The cleavable linker may be enzymatically cleavable. Suitable cleavable linkers are well-known in the art (see e.g. the documents discussed in the background and elsewhere herein, in particular Chen 2013 and Chen 2014) and are also described e.g. in WO2005/084367 and WO2017/139415. If the hybridizing sequencing reagent comprises a photocleavable linker, step b) may comprise photocleaving the linker to release the detectable label from the hybrid. E.g. UV light may be used to photocleave the linker. If the hybridizing sequencing reagent comprises a chemically cleavable linker, step b) may comprise adding a cleave reagent that is suitable to cleave the linker and thereby release the detectable label. The cleave reagent may be comprised in a liquid medium. As discussed, the cleave reagent may be an enzyme if the linker is enzymatically cleavable. The released detectable label may then be separated and e.g. transferred to a detection substrate while being comprised in the liquid medium comprising the cleave reagent. In certain embodiments, the chemically cleavable linker is a disulphide linker. The cleave reagent may comprise a reducing agent. Such technologies are e.g. described e.g. in WO2017/139419 and WO2017/139415 to which it is referred.

According to one embodiment, the hybridizing sequencing reagent comprising a detectable label is a nucleotide analogue comprising a nucleobase, a detectable label and a reversible terminating group. According to one embodiment, the nucleotide analogue is a 3'O-blocked reversible terminator or a 3'unblocked reversible terminator. Such terminators are well known in the art and are e.g. described in Chen 2013, Chen 2014 and Kim, 2014 "Synthesis of 3'-O-fluorescently mono-modified reversible terminators and their uses in sequencing-by-synthesis"; Bioorganic & Medicinal Chemistry Letters Volume 24, Issue 1, Pages 209-213.

According to a preferred embodiment, the nucleotide analogue is a 3'O-blocked reversible terminator which comprises as reversible terminating group a removable moiety capping the 3'-OH group of the nucleotide. Thus, according to a preferred embodiment, the hybridizing sequencing reagent comprising a detectable label is a nucleotide analogue comprising a nucleobase, a detectable label and a removable moiety capping the 3'-OH group of the nucleotide as reversible terminating group. Such nucleotide analogues are commonly used in conjunction with sequencing methods that are based on sequencing by synthesis by cyclic reversible termination. The capping moiety that is used as reversible terminating group, herein also referred to as "cap", prevents extension with another nucleotide in the same sequencing cycle. After the complementary nucleotide analogue has been incorporated into the hybrid and unincorporated nucleotide analogues were removed, the chemical moiety capping the 3'-OH group can be removed. This restores the free 3'-OH group of the nucleotide which thereby becomes available for incorporating a further nucleotide analogue in the next sequencing cycle. In one embodiment, removal of the capping moiety is performed in step b). The capping moiety may be released (and the free 3'OH group restored) in parallel to releasing the detectable label. However, the capping moiety may also be removed prior to or subsequent to releasing the detectable label. The capping moiety may also be released after step c). Suitable capping moieties and suitable conditions to remove the capping moiety are well-known in the art (see e.g. Chen 2013, Chen 2014) and therefore, do not need any detailed description.

According to one embodiment, the removable moiety capping the 3'-OH group is a cleavable moiety capping the 3'-OH group of the nucleotide. Upon cleavage, the 3'OH group can be restored. Details and suitable embodiments are known in the art.

According to one embodiment, the detectable label is attached to the nucleobase of the 3'O-blocked reversible terminator (see e.g. Chen 2013, Chen 2014). This is a commonly used design concept well known in the art. Such embodiments can also be used in conjunction with the invention.

According to one embodiment, the detectable label is bound to the cleavable capping moiety in the 3'O-blocked reversible terminator. Thus, the nucleotide analogue comprising the detectable label may comprise a cleavable moiety capping the 3'-OH group of the nucleotide and the detectable label is bound to the cleavable capping moiety. In this embodiment, the cleavable capping moiety not only blocks the 3'-OH group to prevent the incorporation of a further base after the nucleotide analogue has formed a hybrid with the nucleic acid template, it additionally functions as the cleavable linker in order to attach the detectable label. The reversible terminating group on the 3'OH thus plays a dual role as providing the detectable label as well as reversibly blocking the 3'OH group. During step b), the cleavable capping moiety is cleaved, thereby releasing the cleavable capping moiety to which the detectable label is attached (thereby also releasing the detectable label from the hybrid) and regenerating the 3'-OH group for the next sequencing cycle. The detectable label attached to the capping moiety is thereby released into the liquid medium. Such reversible terminators can be used in conjunction with the present method. Suitable examples for such reversible terminators are e.g. described in Kim, 2014.

According to one embodiment, the hybridizing sequencing reagent comprising a detectable label is a nucleotide analogue comprising a nucleobase and a detectable label, wherein the detectable label is attached to a phosphate. Thus, the detectable label may also be attached to the phosphate of the nucleotide analogue. The nucleotide may comprise e.g. 4 to 6 phosphate residues, wherein the detectable label is attached to the end phosphate group. Such phosphate labelled nucleoside polyphosphates, preferably terminal gamma-phosphate labelled, are known in art. Upon incorporation by the polymerase, the nucleotide is added to the primer terminus and a pyrophosphate (PPi) leaving group is released by the polymerase. The detectable label (e.g. a fluorescent label such as a fluorescent dye) that is covalently attached to the PPi leaving group is thereby released as well. Details are e.g. described in Chen, 2014. To prevent extension after incorporation, the 3'OH group of the nucleotide analogue in which the detectable label is attached to a phosphate is preferably blocked by a capping moiety. Details were described above.

According to one embodiment, step a) comprises contacting the nucleic acid template with at least four nucleotide analogues. According to one embodiment, the first nucleotide analogue is an analogue of A, the second nucleotide analogue is an analogue of G, the third nucleotide analogue is an analogue of T or U, preferably T, and the fourth nucleotide analogue is an analogue of C. As is well-known in the art, to be able to distinguish the four bases that are comprised in a DNA template, it is not required that all four nucleotide analogues comprise a different detectable label. Different labelling formats are known in the art and can be used in conjunction with the present invention. According to one embodiment, at least three of the at least 4 nucleotide analogues comprise a detectable label, i.e. they comprise at least one detectable label. In such embodiments, the bases are distinguishable based on the presence (or absence) of a detectable label. A corresponding technology is e.g. commercially used by Illumina using a 2-color system. E.g. one base (e.g. G) is unlabelled, one base (e.g. C) is labelled red, one base (e.g. T) is labelled green, and the last base (e.g. A) is labelled red and green. This allows to distinguish all four bases in a two-channel system (see e.g. Goodwin, 2016 referring to the NextSeq system). According to one embodiment, each nucleotide analogue comprises a detectable label. According to a preferred embodiment, each nucleotide analogue comprises a different detectable label, preferably a different fluorescent dye. Such 4 color systems are well known in the art and can be used in conjunction with the present method.

According to a preferred embodiment, step a) thus comprises contacting the nucleic acid template with at least four nucleotide analogues each comprising a nucleobase and a detectable label, wherein the first nucleotide analogue is an analogue of A, the second nucleotide analogue is an analogue of G, the third nucleotide analogue is an analogue of T or U, preferably T, and the fourth nucleotide analogue is an analogue of C and wherein the four nucleotide analogues are distinguishable based on the detectable label. As is known in the art, such nucleotide analogues can be used as building blocks for DNA synthesis. Each nucleotide analogue may comprises one of the nucleobases A, T (or U), G and C, a detectable label and a reversible terminating group, e.g. a removable moiety capping the 3'-OH group of the nucleotide.

Typically, the nucleotides are provided as dNTPs (desoxynucleotide triphosphates).

As discussed above, the detectable label is preferably a fluorescent label such as a fluorescent dye. According to one embodiment, each of the four nucleotide analogues has a predetermined fluorescence wavelength which is different than the fluorescence of the other three analogues. The fluorescent dye is accordingly specific for the nucleobase that they are attached to. For example, the four fluorescent dyes may emit blue, green, yellow or red light upon excitation. Unlabelled nucleotide analogues comprising a removable, preferably cleavable, 3'OH capping moiety may be used in addition to the labelled nucleotide analogues. Such methods are also known in the art and are also described in the background of the invention (see e.g. Goodwin, 2016 and GeneReader platform).

Under the conditions used in step a) the nucleotide analogue complementary to the base of the nucleic acid template is bound to the nucleic acid template thereby providing the hybrid comprising the detectable label. Suitable conditions are well-known in the art and involve e.g. the addition of a polymerase and optionally a primer. As is known in the art, the nucleotide analogue may form a phosphodiester bond with the 3' end of a nucleic acid primer that is hybridized to the nucleic acid template or the 3'end of the nucleic acid template if a self-priming nucleic acid template is used. Thereby, a hybrid is provided which comprises the incorporated nucleotide analogue comprising the detectable label which is complementary to the residue to be identified. It is standard to use a polymerase to catalyse this reaction. Suitable polymerases are known in the art and are also described e.g. in Chen, 2014. The reversible terminating group, which preferably is a 3'OH capping moiety, prevents the incorporation of a further nucleotide analogue during the same sequencing cycle. Unincorporated nucleotide analogues are then removed and a wash step may be performed. In subsequent step b), the incorporated detectable label is then released from the hybrid. In certain embodiments, the capping moiety is also removed in the course of step b). However, it is also within the scope of the present invention to remove the capping moiety in a separate step. Such separate step may be performed prior to or after step b) and may also be performed after step c). In step c), the released detectable label is separated from the nucleic acid template prior to detecting the separated detectable label in step d). Details and specific embodiments are also described in the example section.

The method according to the present invention preferably comprises performing repeated sequencing cycles comprising steps a) to c) in order to determine the sequence of the nucleic acid template. The number of sequencing cycles required to determine the sequence of a nucleic acid template depends on the length of the nucleic acid template and/or the desired read length. In one embodiment, one base of the nucleic acid sample is identified per sequencing cycle.

In one embodiment, Xn sequencing cycles comprising steps a) to c) are performed, wherein preferably n is at least 10. In embodiments, n is at least 20, at least 25, at least 50 or at least 75. In advantageous embodiments, n is at least 100, at least 125 or at least 150. However, depending on the chosen design also more cycles of at least 200, at least 250 or at least 300 are feasible. In embodiments, n is selected from 25 to 1000, 50 to 750, 75 to 750, 100 to 500 and 125 to 250.

In one embodiment, the method comprises performing repeated sequencing cycles comprising steps a) to c) before detecting the detectable labels separated in the sequencing cycles for determining the sequence of the nucleic acid template.

In one embodiment, detecting the detectable label separated in a sequencing cycle occurs independently and in parallel to further sequencing cycles performed for determining the sequence of the nucleic acid template.

Separating the released detectable label in step c) may involve transferring released detectable label (e.g. in a liquid, preferably aqueous medium) to a detection substrate and detecting may comprise moving the detection substrate with the transferred detectable label to an optical device comprising a sensor. The transferred detectable label preferably forms at least one spot on the detection substrate. The sensor may scan the spot to detect the transferred detectable label, thereby allowing to determine the residue of the nucleic acid template of the corresponding sequencing cycle.

### Nucleic acid template

Nucleic acid templates for sequencing as well as their preparation are well known in the art and therefore, do not need to be described herein in detail. As is described in the background of the invention, common steps for preparing the nucleic acid template for sequencing usually include steps such as nucleic acid isolation from a biological sample, library preparation, target enrichment and clonal amplification. Such steps may be performed in the method of the present invention in order to prepare the nucleic acid template for sequencing.

According to one embodiment, the nucleic acid template is a DNA template. The DNA template may have been prepared from RNA by reverse transcription. The nucleic acid template may be at least partially double-stranded. According to one embodiment, the nucleic acid template is a self-priming template. The nucleic acid template may comprise multiple copies of the same strand to be sequenced. The nucleic acid template may comprise sequencing adaptors as are commonly used in the art. The nucleic acid template is at least partially single stranded, at least during the sequencing process, to allow the hybridization of the hybridizing sequencing reagent comprising a detectable label. The DNA template may have a length in a range selected from 50 to 1000, 50 to 800 and 50 to 500 nucleotides. Suitable sizes can be obtained by fragmentation. Suitable techniques are well-known in the art.

In one embodiment, the nucleic acid template is provided as nanoball, also referred to as rolony (Rolling Circle Amplified Colony). Methods for providing DNA nanoballs are well-known in the art and therefore, do not need to be described in detail. In an embodiment for generating DNA nanoballs, the DNA (after optional fragmentation to obtain suitable fragment sizes for sequencing) is ligated to a first of four adapter sequences. The template is amplified, circularized and cleaved with a type II endonuclease. A second set of adapters is added, followed by amplification, circularization and cleavage. This process is repeated for the remaining two adapters. The final product is a circular template with four adapters, each separated by a template sequence. The molecules undergo a rolling circle amplification step, generating a large mass of concatamers referred to as DNA nanoballs. DNA nanoball sequencing has the advantage of achieving a high density. DNA nanoballs can be sequenced by sequencing by ligation (see e.g. Goodwin, 2016) as well as sequencing by synthesis (see e.g. Xu, Mingya, "Nex-Generation Sequencing for Biomedical Applications" (2013), Biomedical Sciences ETDs, Paper 152). DNA nanoballs may be immobilized to a surface, e.g. in form of an array. Suitable immobilization techniques are known in the art.

According to one embodiment, the nucleic acid template is immobilized in a sample zone at least during step c), preferably during steps a) to c). This simplifies the separation of released detectable label from the nucleic acid template in step c). As is described herein, such sample zones may be provided e.g. in the reservoir of a printing device or on a sample substrate. According to one embodiment, multiple copies of the same nucleic acid template are immobilized to the same sample zone. This advantageously increases the sensitivity. In embodiments, different nucleic acid templates may be immobilized in different sample zones of a device. The sample zones may be arranged as array. Exemplary embodiments are described herein.

As is known in the art, the nucleic acid template to be sequenced may be immobilized to a surface. In a preferred embodiment, the nucleic acid template is provided to a sample zone provided on a support (e.g. the reservoir or nozzle of a printing device or a sample substrate) and immobilized in the sample zone. Immobilization of the nucleic acid template to the surface, respectively the sample zone may be e.g. achieved covalently or by other bonding mechanisms, such as electrostatic or hydrophobic interactions. Details are known in the prior art. In one embodiment, the nucleic acid template is covalently attached to the sample zone. Suitable embodiments for immobilizing a nucleic acid template are known in the art and are e.g. used in sequencing methods to immobilize nucleic acid templates on a flow cell, e.g. a patterned flow cell, or other surfaces using appropriate immobilization chemistry. The nucleic acid template can be immobilized in the sample zone by well-known substrate functionalization technologies based on inorganic or organic biofunctionalization moieties or agents. The nucleic acid template can be covalently attached to a surface, if the surface and the nucleic acid template comprise chemical moieties which can be covalently linked. Such methods are known in the art and applicable corresponding groups could be amine and carboxyl. In a preferred embodiment, the surface to which the nucleic acid template is supposed to be attached (e.g. a sample zone) has been activated, i.e. has been equipped with a reactive group for specifically binding the nucleic acid template. Methods are known in the art for activating surfaces and making them suitable for covalent attachment of nucleic acid molecule. E.g. functionalized polymers can be used for attachment. The polymer may be a polymeric or polymer-precursor material carrying at least one functionality for specifically or non-specifically attaching the nucleic acid template via electrostatic interaction, hydrogen-bond interaction or covalent linkage. Suitable chemical functionalities for attachment are known to the person skilled in the art, and include but are not limited to functional groups such as epoxides, aldehydes, activated carboxylates, amines, azides, thiols, amides, hydrazides and isothiocyanates. According to one embodiment, polymer provides an amino function for coupling. For the specific case of amino functions, polymeric materials might include poly-L-lysine, polyallylamine, polyethylenimine or polymer precursors such as aminopropyltrimethoxysilane. In one embodiment, the surface comprises azide groups, to which the nucleic acid template is directly or indirectly attached.

According to one embodiment, the nucleic acid template is immobilized on a carrier, such as a particle, preferably a magnetic particle. Preferably, multiple copies of the same nucleic acid template are immobilized on the same magnetic particle. Suitable embodiments for providing according magnetic particles are known in the art. The magnetic particle(s) comprising the same nucleic acid template may be immobilized to the same sample zone by the aid of a magnetic field.

### Use of a printing device for transferring released detectable label

According to an advantageous embodiment, the released detectable label is comprised in a liquid medium and the method comprises printing released detectable label through the nozzle of a printing device to a bypass-location, such as a detection substrate. Advantageously, the use of a printing device allows to transfer released detectable label that is comprised in a liquid medium in an efficient and fast manner to a bypass-location, such as a detection substrate.

In one embodiment, released detectable label comprised in a liquid medium is provided to a reservoir of a printing device. The reservoir can serve as container. The released detectable label may be provided in the reservoir, after it has been obtained and before it is transferred through the printer nozzle to the detection substrate. The reservoir may form an integral part of the printing device and may form part of the nozzle. In one embodiment, the reservoir is a receptical that can be detached from the printing device. The nozzle may be e.g. of circular or of elliptical shape.

In one embodiment, released detectable label comprised in a liquid medium is provided to the reservoir from a different location, for example from a reaction chamber, where step a) and optionally step b) have been performed. Released detectable label is then printed from the reservoir onto the detection substrate. Hence, step a) and step b) may be carried out in a chamber separate from the reservoir of the printing device. The released detectable label comprised in a liquid medium is then transferred to the reservoir of the printing device before it is printed. The chamber in which steps a) and b) were carried our may be in fluid connection with the reservoir of the printing device. Alternatively, the liquid medium comprising the released detectable label may also be transferred to the reservoir of the printing device using a pipetting apparatus or a second printing device.

According to a preferred embodiment, a printing device is used in order to assist the separation of released detectable label from the nucleic acid template. Separating released detectable label from the nucleic acid template in step c) may thus involve transferring released detectable label to a bypass-location, such as a detection substrate, by use of a printing device. Hence, according to a preferred embodiment of the method according to the present invention, the released detectable label is comprised in a liquid medium and separating in step c) involves transferring released detectable label to a detection substrate by printing released detectable label comprised in a liquid medium through the nozzle of a printing device onto the detection substrate. The detection substrate is preferably spaced apart from the printing device. Details of the detection substrate are discussed elsewhere herein and it is referred to the respective disclosure. The method provides the advantage that the spots created by the transfer of the released detectable label to the detection substrate through the printer nozzle can dry out after printing. E.g. the detection of dry fluorophores is possible. Therefore it is not needed to prevent evaporation of the transferred droplet on the detection substrate.

In a preferred embodiment, steps a) and/or b), preferably steps a) and b), are performed within the printing device, e.g. within a reservoir. The reservoir may also be provided by or within a nozzle. The detectable label that is released in step b) into a liquid medium comprised in the reservoir of the printing device can then be printed from the reservoir through the opening of the nozzle onto a detection substrate, whereby it is separated from the nucleic acid template. In one embodiment the detection substrate is kept stationary after released detectable label has been printed onto the detection substrate, the location on the detection substrate onto which the released detectable label has been printed hence forming the bypass-location. In such an embodiment, the printing device or elements thereof can, for example, be moved away from the detection substrate to prevent any interference between products dripping from the printing device after performance of the printing or products being discharged from the printing device in later method steps with the released detectable label having been printed onto the detection substrate. In a further embodiment, the detection substrate is moved after the released detectable label has been printed onto the detection substrate.

According to one embodiment, the method comprises
- providing a printing device, wherein the nucleic acid template is comprised in the printing device, and
- performing steps a) and b) in the printing device, and
- wherein step c) comprises printing released detectable label comprised in a liquid medium onto a detection substrate.

As discussed herein, the printing device preferably comprises a reservoir and a nozzle, i.e. at least one nozzle, wherein optionally, the reservoir is provided by or within the nozzle.

According to a preferred embodiment, the method according to the present invention comprises
- providing a printing device comprising a reservoir and a nozzle, wherein the nucleic acid template is comprised in the reservoir, and
- performing steps a) and b) in the reservoir, and
- wherein step c) comprises separating released detectable label from the nucleic acid template by transferring released detectable label comprised in a liquid medium through the nozzle onto a detection substrate. Preferably, the detection substrate is spaced from the printing device. The detection substrate comprising transferred detectable label is then used to detect and identify the released detectable label in step d). The reservoir may be provided by or within a nozzle.

Released detectable label comprised in a liquid medium is preferably printed in a definite location onto the detection substrate. The printed detectable label preferably forms at least one spot on the detection substrate. Printing is advantageously performed in an ordered, preferably patterned manner, e.g. an array, thereby allowing to correlate the printed spots with the nucleic acid template(s) comprised in the reservoir of the printing device. This is particularly advantageous if multiple nucleic acid templates are processed in parallel. Embodiments are described herein.

The method may comprise performing repeated sequencing cycles Xn comprising steps a) to c), wherein the method comprises
- providing a printing device comprising a nozzle, wherein the nucleic acid template is comprised in the printing device, preferably in a reservoir,
- performing a first sequencing cycle X1 comprising
   a1) contacting within the printing device the nucleic acid template with a sequencing reagent comprising a detectable label under suitable conditions to provide a hybrid comprising a detectable label;
   b1) releasing the detectable label from the hybrid into a liquid medium comprised in the printing device; and
   c1) separating released detectable label from the nucleic acid template that is comprised in the printing device wherein separating comprises transferring released detectable label comprised in the liquid medium through the nozzle onto a detection substrate, thereby providing at least one spot comprising the detectable label released during the first sequencing cycle on the detection substrate;
- performing a second sequencing cycle X2 comprising
   a2) contacting within the printing device the nucleic acid template obtained after the first sequencing cycle with a sequencing reagent comprising a detectable label under suitable conditions to provide a hybrid comprising a detectable label;
   b2) releasing the detectable label from the hybrid into a liquid medium comprised in the printing device; and
   c2) separating released detectable label from the nucleic acid template that is comprised in the printing device wherein separating comprises transferring released detectable label comprised in the liquid medium through the nozzle onto a detection substrate, thereby providing at least one spot comprising the detectable label released during the second sequencing cycle on the detection substrate;
- performing further subsequent sequencing cycles Xn comprising steps an) to cn), wherein step cn) of each further sequencing cycle comprises separating released detectable label from the nucleic acid template that is comprised in the printing device wherein separating comprises transferring released detectable label comprised in a liquid medium through the nozzle onto a detection substrate, thereby providing as result of each subsequent sequencing cycle at least one transferred spot comprising the detectable label released during each subsequent sequencing cycle on a detection substrate;
wherein the method further comprises detecting the spotted detectable labels for determining the sequence of the nucleic acid template

According to a preferred embodiment, the method comprises performing repeated sequencing cycles Xn comprising steps a) to c), wherein the method comprises
- providing a printing device comprising a reservoir and a nozzle, wherein the nucleic acid template is comprised in the reservoir,
- performing a first sequencing cycle X1 comprising
   a1) contacting within the reservoir the nucleic acid template with a sequencing reagent comprising a detectable label under suitable conditions to provide a hybrid comprising a detectable label;
   b1) releasing the detectable label from the hybrid into a liquid medium comprised in the reservoir; and
   c1) separating released detectable label from the nucleic acid template that is comprised in the reservoir wherein separating comprises transferring released detectable label comprised in the liquid medium through the nozzle onto a detection substrate, thereby providing at least one spot comprising the detectable label released during the first sequencing cycle on the detection substrate;
- performing a second sequencing cycle X2 comprising
   a2) contacting within the reservoir the nucleic acid template obtained after the first sequencing cycle with a sequencing reagent comprising a detectable label under suitable conditions to provide a hybrid comprising a detectable label;
   b2) releasing the detectable label from the hybrid into a liquid medium comprised in the reservoir; and
   c2) separating released detectable label from the nucleic acid template that is comprised in the reservoir wherein separating comprises transferring released detectable label comprised in the liquid medium through the nozzle onto a detection substrate, thereby providing at least one spot comprising the detectable label released during the second sequencing cycle on the detection substrate;
- performing further subsequent sequencing cycles Xn comprising steps an) to cn), wherein step cn) of each further sequencing cycle comprises separating released detectable label from the nucleic acid sample that is comprised in the reservoir wherein separating comprises transferring released detectable label comprised in a liquid medium through the nozzle onto a detection substrate, thereby providing as result of each subsequent sequencing cycle at least one transferred spot comprising the detectable label released during each subsequent sequencing cycle on a detection substrate;
wherein the method further comprises detecting the spotted detectable labels for determining the sequence of the nucleic acid template.

As is described herein, separating released detectable label from the nucleic acid template in step c) can be performed by transferring released detectable label comprised in a liquid medium through the nozzle that is comprised in or connected to the reservoir onto a detection substrate. The liquid medium is preferably an aqueous medium. It can be provided by the liquid medium that is used in step b) to release the detectable label from the hybrid.

As disclosed herein, the method according to the present invention may be used for sequencing by synthesis by cyclic reversible termination. After the first sequencing cycle has been performed, the nucleic acid template that is obtained after the first sequencing cycle is in one embodiment the hybrid that was obtained in the first sequencing cycle and from which the detectable label and the capping moiety has been removed. As discussed above, in a preferred embodiment wherein labelled nucleotide analogues are used as hybridizing sequencing reagent, each sequencing cycle allows to identify a single base of the nucleic acid template based on the detectable label that is incorporated by the nucleotide analogue that is capable of hybridizing with the corresponding base of the nucleic acid template, whereby a hybrid is provided that is extended by a single base, namely the incorporated nucleotide analogue. The resulting hybrid that is extended by a single base is in the next sequencing cycle again contacted with labelled nucleotide analogues to incorporate the next labelled nucleotide analogue that is capable of hybridizing with the next base of the nucleic acid template. The details and embodiments of the sequencing reaction are well known in the art and are also described in the background of the invention and elsewhere herein.

In one embodiment of the method wherein repeated sequencing cycles Xn comprising steps a) to c) are performed, the released detectable labels of at least two or all sequencing cycles are transferred onto the same detection substrate, whereby a detection substrate is provided that comprises spots comprising the detectable label released during two or more or all sequencing cycles. E.g. transferring all released detectable labels onto the same detection substrate allows to determine the sequence of a nucleic acid template by analysis of a single detection substrate. As discussed above, this advantageously reduces the detection steps required in order to identify the released detectable labels and hence to determine the sequence of the nucleic acid template. Instead of identifying the released detectable label in the course of each sequencing cycle (which e.g. requires taking 4 images per base when using a fluorescent label), all released detectable labels can be identified subsequently by analysis of the obtained detection substrate which comprises all released detectable labels. This allows to simplify the handling and to reduce the turn-around-times. Moreover, as discussed above, significantly less images are required for determining the sequence as several labels and thus bases can be detected together.

In a further embodiment of the method wherein repeated sequencing cycles Xn comprising steps a) to c) are performed, the released detectable labels of at least two or all sequencing cycles are transferred onto different detection substrates. According to one embodiment, a provided detection substrate comprises the detectable label released during a single sequencing cycle of a nucleic acid template. An according detection substrate thus provides information on the sequence portion obtained in a single sequencing cycle, e.g. a single base, of the nucleic acid template to be sequenced. As is disclosed herein, the detection of the detectable label and thus the identification of the corresponding sequence portion, e.g. a single base, can be advantageously performed independently of the further sequencing cycles when using the method of the present invention. This allows to perform the detection of the detectable label and thus the identification of the corresponding sequence portion in parallel to further sequencing cycles. E.g. the detection substrate comprising the released detectable label from the first sequencing cycle can be transferred to a detection device for detection, while the next sequencing cycle is performed in parallel. This also allows to shorten the turn-around-times. This embodiment is e.g. advantageous if multiple nucleic acid templates are processed in parallel using a printing device comprising multiple printing units. The printing device can print the detectable labels released during a single sequencing cycle of the different nucleic acid templates through the nozzles of the different printing units onto the same detection substrate. As disclosed herein, an according printing device may comprise e.g. several thousand printing units. Accordingly, the detectable labels released during a sequencing cycle of several thousand nucleic acid templates can be detected, while the next sequencing cycle(s) of the several thousand nucleic acid templates is performed in parallel. This also allows to shorten turn-around-times.

The method may furthermore comprise performing one or more additional method steps in the reservoir of the printing device. Such method step(s) can be performed e.g. to prepare the nucleic acid template for the next sequencing cycle and such steps may also be performed inbetween sequencing cycles. Furthermore, such method step(s) may also be performed in advance to prepare or provide the nucleic acid template for sequencing. In embodiments, one or more amplification steps are performed in the reservoir of the printing device to provide the nucleic acid template. E.g. the printing device can be part of the seeding and/or amplification process of the nucleic acid template, e.g. rolling circle amplification (RCA) if the nucleic acid template is provided as DNA nanoball. These processes may be performed in the reservoir of the printing device.

The reservoir of the printing device can be heated and/or cooled according to the needs of a reaction performed in the reservoir, e.g. one or more steps of the sequencing reaction. The heating and cooling can be performed in sequence during the performance of the reaction.

As discussed, according to one embodiment, step a) is performed in the reservoir of the printing device. According to this preferred embodiment, the nucleic acid template may be provided in the reservoir of the printing device and the hybridizing sequencing reagent comprising the detectable label (e.g. in form of a mixture of differently labelled nucleotide analogues or in form of a mixture of differently labelled oligonucleotide probes) and further reagents required to establish suitable conditions for generating the hybrid in step a) are added. Preferably, the reagents are added in form of one or more liquids. In one embodiment the reactive chemistry required for performing step a) is provided as liquid from a container. The container may be placed above the printing device in a way that it is possible to add the reactive chemistry from the container by letting it simply fall into the reservoir of the printing device. For this purpose, the printing device may have an opening for receiving the liquid medium comprising the reactive chemistry for performing step a). Preferably, the opening can be closed after adding the liquid medium. The container may be provided in form of a second printing device or dispenser with a nozzle. The liquid medium comprising the reactive chemistry for performing step a) may also be provided to the reservoir of the printing device by way of a tube that opens into the reservoir. In a preferred embodiment, steps a) and b) of the method are carried out in the reservoir of the printing device. After step a) has been completed and the hybrid comprising the incorporated detectable label has been provided, the remaining reaction fluid of step a) is removed. This can be done e.g. by discharging the remaining fluid from the reservoir into a waste container. Discharging may occur through the nozzle. The hybrid may be further processed prior to performing step b), e.g. it may be washed to remove unincorporated hybridizing reagent comprising a detectable label. Washing fluid is then removed, e.g. discharged, from the reservoir. During step b) the detectable label is released from the hybrid. Suitable embodiments are described herein. The detectable label is preferably released into a liquid medium. Suitable methods to release the detectable label *inter alia* depend on the hybridizing sequencing reagent used. As is discussed herein, in some SBL embodiments (e.g. used for DNA nanoball sequencing) that can be used in conjunction with the present method the entire labelled probe that was incorporated in step a) is subsequently removed, thereby likewise releasing the incorporated label (attached to the probe) from the hybrid and restoring the nucleic acid template for the next sequencing cycle. The released labelled probe is then be separated from the nucleic acid template as is described herein prior to detecting the separated detectable label. Further embodiments are also described herein. According to one embodiment, the hybridizing sequencing reagent comprises a cleavable linker to which the detectable label is attached. The cleavable linker is accordingly present in the hybrid that is formed in step a). Step b) may thus comprise adding to the reservoir a liquid medium comprising one or more reagents suitable to cleave the cleavable linker, thereby releasing the detectable from the hybrid through cleavage of the linker. The released detectable label floats then in the liquid medium. In case a nucleotide analogue is used which comprises a removable moiety capping the 3'-OH group of the nucleotide, the step of removing the capping moiety may also be performed in the reservoir of the printing device. The step of removing the capping moiety may be performed after unincorporated nucleotide analogues were removed prior to, during or subsequent to step b). Suitable embodiments are described herein.

According to one embodiment, the nucleic acid template is immobilized in the reservoir of the printing device at least during step c), preferably during steps a) to c). The reservoir of the printing device may comprise one or more sample zones, in which nucleic acid templates can be immobilized. The one or more sample zones can be, for example, located at a sidewall that delimits the reservoir of a printing device. As discussed in a general context above, different nucleic acid templates may be immobilized in different sample zones. The sample zones may be arranged in form of an array. This allows to process different nucleic acid templates in parallel. Furthermore, the printing device may comprise multiple distinct reservoirs, wherein each reservoir determined to receive a nucleic acid template to be sequenced comprises or is connected to an individual nozzle. The nozzle allows to transfer released detectable label to a detection substrate. Different nucleic acids may be immobilized to a sample zone in different reservoirs.

The nucleic acid template may be immobilized to the sample zone of a reservoir by using appropriate immobilization chemistry. The nucleic acid sample may be covalently attached. Suitable immobilization techniques are known in the art and are also described herein. Furthermore, the nucleic acid template may be immobilized to a carrier, preferably a magnetic carrier. The magnetic carrier may be a magnetic particle such as a magnetic bead. Suitable methods are well-known in the art and widely used. The magnetic carrier carrying the nucleic acid template, such as e.g. a magnetic bead, may then be immobilized in the sample zone of the reservoir of the printing device by application of a magnetic field. As discussed above, it is preferred to immobilize multiple copies of the same nucleic acid template in a sample zone comprised in a reservoir.

In a preferred embodiment, the transfer of the released detectable label to the bypass location, e.g. onto a detection substrate, includes the creation of droplets of the liquid medium comprising the released detectable label in a nozzle and the transfer of the droplets to a detection substrate, for example by way of letting the droplets drop down or by way of propelling the droplets by use of an electric field. The droplets are transferred to the detection substrate and thereby form one or more spots on the detection substrate. The creation of droplets may be stopped once the desired amount of liquid medium comprising the released detectable label has been printed onto the detection substrate. Liquid remaining in the reservoir and/or the nozzle of the printing device may then be removed, e.g. by discharging remaining liquid into a waste chamber. Furthermore, the reservoir comprising the nucleic acid may be subsequently washed and/or the nucleic acid template may be further treated, e.g. with a liquid media, before step a) of the next sequencing cycle is again performed.

According to one embodiment the reservoir of the printing device is provided by a section of a longitudinal trough. The reservoir may be provided by the section of a longitudinal trough that borders at least one nozzle arranged at the bottom of the trough.

In one embodiment, where the reservoir is provided by a section of a longitudinal trough a fluid required to perform steps a) and optionally further steps is brought into contact with the nucleic acid template in the reservoir by way of letting said fluid flow along the trough. By controlling the flow velocity of the fluid the time of contact between the nucleic acid template and the fluid can be influenced. E.g. the flow of the fluid along the trough can be interrupted and the reaction fluid kept stationary for a period of time, while it is in contact with the nucleic acid template. According to one embodiment, different fluids are directed to flow along the trough in the course of performing the method according to the present invention. Undesired contaminations between different fluids can be avoided by ensuring that each fluid is separated from each other by an inert fluid. In this embodiment, an inert fluid refers to a fluid that does not contribute to the sequencing reaction. Examples include but are not limited to wash fluids or fluids immiscible in water, such as oil. These inert fluids may function as a liquid plug between reaction fluids. A fluid comprising reactive chemistry, also referred to as reaction fluid herein, refers to a fluid that contributes to the sequencing reaction. Examples include (i) the fluid comprising the hybridizing sequencing reagent comprises a detectable label (e.g. labelled nucleotide analogues) and one or more enzymes (e.g. polymerase) and optionally further reagents (e.g. primers), (ii) a fluid comprising the reactive chemistry for releasing the detectable label from the hybrid and/or (iii) a fluid comprising the reactive chemistry for removing the capping moiety. The flow of a reaction fluid along the trough may thus be controlled by neighbouring inert fluids which function as liquid plugs. They are arranged at either side of a reaction fluid to control its position and distribution along the trough. In one embodiment, a sequence of reaction fluids is provided in the course of the sequencing reaction, each reaction fluid being separated from the next reaction fluid by an inert liquid. In such an arrangement, the sequence of the first reaction fluid, inert fluid, second reaction fluid, inert liquid (etc.) can be made to flow along the trough and hence through the reservoir and in doing so can be brought into contact with the nucleic acid template sequentially. In one embodiment, the trough contains several printing units, here in the form of printing zones. This can be achieved by providing the trough with several nozzles at the bottom of the trough arranged distanced from one another in the direction of the extension of the trough. Each printing zone is associated with a nozzle. In such an embodiment, a reaction fluid can be made to flow through several printing units, respectively printing zones, in sequence. This allows for the method to be conducted on several nucleic acid templates in parallel. The trough may be patterned to structure the printing zones. Each printing zone preferably comprises a sample zone. The nucleic acid template may be immobilized to the sample zone of a printing unit, respectively printing zone. Details of this embodiment are also disclosed elsewhere herein. In one embodiment, the sample zone is hydrophilic and is surrounded by hydrophobic areas. Several reaction fluids (e.g. for performing step a), wash fluids and e.g. fluids for releasing the reversible terminating group, if not at the same time releasing the detectable label) may flow through the trough as described herein. However, the reaction fluid comprising the chemistry for releasing the detectable label should be applied to the sample zone in a way that cross-contamination of released detectable label from one sample zone with released detectable label of neighbouring sample zones is avoided. This can be achieved e.g. if applying the reactive chemistry for releasing the detectable label in step b) to each sample zone provided in the trough while avoiding contact with other sample zones comprising different nucleic acid templates. This can be achieved e.g. by using a second printing device which applies the reactive chemistry for releasing the detectable label to the individual sample zones provided in the trough. The released detectable label comprised in a liquid can then be separated from the nucleic acid template immobilized to the sample zone by printing one or more droplets comprising the released detectable label through the nozzle of the corresponding printing zone. To prevent cross-contamination, hydrophobic regions may be provided between the hydrophilic sample zones, respectively the printing zones. Other embodiments are also feasible.

### Exemplary details of the printing device

As discussed above, according to one embodiment the released detectable label is transferred to a bypass-location, e.g. a detection substrate, using a printing device. In a preferred embodiment, where the released detectable label comprised in a liquid medium is present in a reservoir of a printing device (e.g. by way of steps a) and b) having been carried out in the reservoir or by way of steps a) and b) having been carried out in a chamber separate from the reservoir and the released detectable label having been transferred to the reservoir), the released detectable label may be printed onto a detection substrate as part of the transfer of the released detectable label to the bypass-location.

The printing device may be or comprise a common ink-jet printhead and can for example make use of a piezo-, acoustic- or thermal actuation of the liquid in order to create a drop out of a meniscus of a liquid arranged in the nozzle and have the created drop being urged to travel away from the meniscus, preferably to travel from the meniscus towards the detection substrate. Such printing devices allow for placing liquid onto a substrate in spots that have the diameter of the order of magnitude of e.g. 10 micrometers or less. For DNA sequencing, especially for the identification of individual bases of the DNA, spots that have a diameter of less than 10 µm, less than 5µm, less than 3µm, less than 2µm, less than 1µm , less than 0.8 µm, less than 0.6µm, less than 0.2µm or less than 0.15µm are advantageous. This reduces the amount of reagents needed for sequencing. Furthermore, more spots can be arranged on given detection substrate which is advantageous for high throughput applications.

According to one embodiment, the released detectable label comprised in a liquid medium is discharged as one or more droplets from the nozzle onto the detection substrate to form a spot in a definite location on the detection substrate. According to one embodiment, the nozzle of the printing device does not contact the detection substrate during the printing process. Hence, in a preferred embodiment the released detectable label comprised in a liquid medium is transferred to a detection substrate which is spaced from the printing device. In such an embodiment, the released detectable label exits the printing device through the nozzle. Then, the detectable label comprised in a liquid medium moves through the space between the printer nozzle and the detection substrate before being applied on the detection substrate.

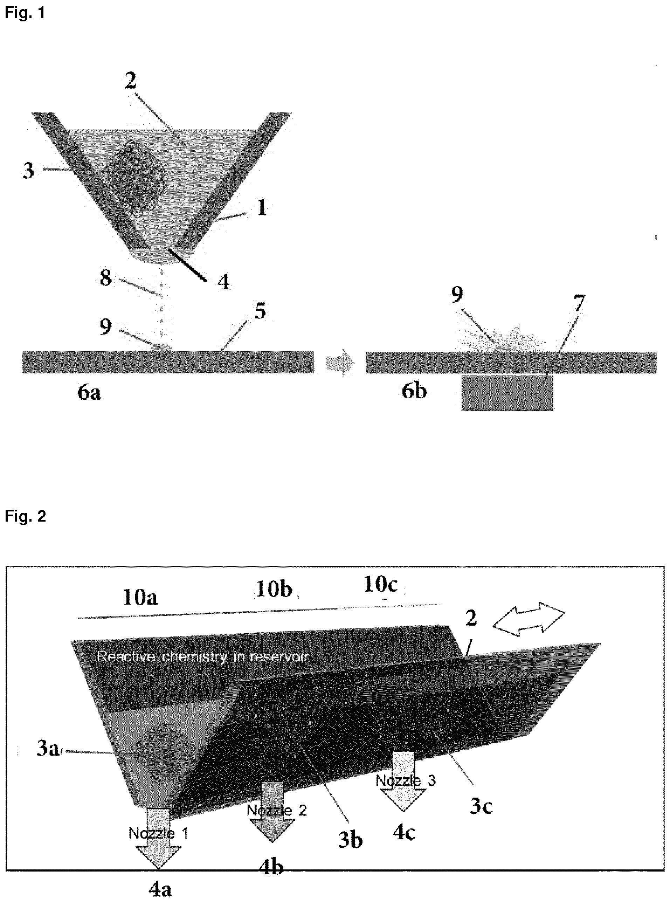

In one embodiment, the printing device is suitable to create a drop out of a meniscus of a liquid arranged in the nozzle and have the created drop being urged to travel from the meniscus towards the detection substrate, if the liquid comprising the released detectable label is placed into the nozzle. The reservoir of the printing device is in fluid-communication with the nozzle such that a fluid in the reservoir can flow into the nozzle and create a meniscus in the nozzle. In a preferred embodiment, the printing device is an electrohydrodynamic printing device. In a preferred embodiment, the printing device is an electrohydrodynamic printing device of the type described in EP 1 477 230 A1, corresponding WO 2013/000558 or WO2016/12081 to which it is referred for details regarding the printing devices that can be used in conjunction with the method according to the present invention. In a preferred embodiment, the printing device is an electrohydrodynamic printing device of the type described in EP 2 540 661 A1 and WO2013/000558. The text portions and Fig. of EP 2 540 661 A1 that describe the design of a printing device, especially the sections [0012] to [0075] and the Fig. 1 and Fig. 20 being incorporated into this description by reference as description of the possible design principle of a printing device that can be used in the context of the present invention. The text portions and Fig. of EP 2 540 661 A1 that describe the relationship of the printing device relative to the substrate, especially the sections [0012] to [0075] and the Fig. 1 and Fig. 20 being incorporated into this description by reference as description of the possible design of the relationship between printing device and the substrate according to the invention.

The printing technology used in an electrohydrodynamic printing device allows ultra-high resolution inkjet printing with nanoscale droplets. Nanodripping is particularly advantageous. As is described in detail in EP 2 540 661, nanodripping allows producing monodisperse droplets smaller than 100 nm at frequencies exceeding 100 kHz. The charged droplets are generated through an electric field that allows precise control of droplet flight and contact-free droplet application to the detection substrate. Advantageously, the droplet generation is fast when using according printing devices. It can be in the range of 1 to 100 cm² per second and the patterning resolution is very high. The droplets can have a diameter which can be much smaller than the meniscus diameter with the latter being directly related to the geometrical properties of the nozzle. It allows to save reagent costs through the generation and printing of tiny droplets of reagents that are tailored to the interaction with the DNA template. The droplets can be e.g. in the nanoliter, picoliter, femtoliter, attoliter or zeptoliter range. The use of this printing technology in order to transfer the released detectable label in a liquid medium to the detection substrate therefore has important advantages. Furthermore, as is also described herein, this printing technology can also be advantageously used to apply liquid fluids to a nucleic acid template that is immobilized to a sample substrate, preferably in a discrete sample zone. As discussed herein, this allows to reduce the amount of reagents required and thereby allows to save costs. Moreover, it allows the design of sequencing systems having a lower dead volume.

Hence, according to one embodiment a printing device is used which allows to discharge droplets out of a liquid continuum by applying an electric field. The electric field can be used to control the flight trajectory of the droplet onto the detection substrate. This allows as explained contactless printing of liquids with an ultra-high resolution and at extremely low liquid volumes. This high resolution allows to locate many droplets on a small surface area (high density), thereby allowing to form small, discrete spots. This is highly advantageous for massively parallel sequencing. The electric field may also be used to adjust the size of the charged droplets. The electric field can be furthermore used to eject all liquid reagents from the reservoir by a jetting impulse if required. This is advantageous to efficiently release e.g. waste liquids.

According to one embodiment, the generated droplets have a diameter of between 1 µm and 0.1 µm, preferably between 0.5µm and 0.1µm (nanodroplets). Alternatively, other sizes are possible, for example micro-sized droplets (with a diameter of 1µm to 10µm), depending on the desired spot size. Other shapes than droplets such as liquid jets are alternatively possible, too.

In a preferred embodiment, the printing device accordingly has an electrode that is suitable to come into contact with the liquid, if the liquid is placed into the nozzle. The electrode can be provided by way of a coating, preferably a metallic coating of at least a part of the wall that delimits the nozzle. According to one embodiment, the electrode is electrically coupled to a wiring of a control circuit of the printing device. Preferably the counter-electrode is electrically coupled to a wiring of a control circuit of the printing device. Preferably, the control circuit of the drive head has a source of electrical power. Preferably the control circuit can apply a potential difference across the electrode and the counter-electrode. This potential difference is sufficient so that the charged droplets are transferred to the detection substrate. This can be triggered and controlled by a computer. In a preferred embodiment the counter-electrode is in contact with the detection substrate. The counter-electrode preferably is positioned in or/and on and/or below and/or above the detection substrate. Details are described e.g. in EP 1 477 230 A1 and corresponding WO 2013/000558 and it is also referred to WO2016/12081.

According to one embodiment, the method comprises using a printing device comprising a multitude of nozzles. This embodiment is advantageous for high-throughput applications as it allows the processing of multiple different nucleic acid templates in parallel. Detectable labels released during the sequencing of different nucleic acid templates may be discharged through the different nozzles (e.g. one nozzle per nucleic acid template) in order to print the detectable label released during an individual sequencing cycle onto a detection substrate. Different designs of the printing device comprising multiple nozzles are feasible and can be used in conjunction with the present invention.

According to one embodiment, the method is performed using a printing device comprising a multitude of printing units wherein each printing unit comprises or is connected to a nozzle. A printing unit may comprise a sample zone to which the nucleic acid template is or can be immobilized. The printing device may comprise an array of printing units.

According to one embodiment, the printing device comprises different nucleic acid templates in different printing units, wherein each printing unit comprises or is connected to a nozzle.

According to one embodiment, the method is performed using a printing device comprising an arrangement, e.g. an array of printing units, each printing unit comprising or being connected to a nozzle and comprising a nucleic acid template to be sequenced, wherein steps a) to c) are performed in parallel in each printing unit comprising a nucleic acid template and wherein step c) comprises transferring released detectable label comprised in a liquid medium through each nozzle onto a detection substrate. The detectable labels released during a sequencing cycle are thus printed in parallel from the printing units onto the detection substrate. The nucleic acid template remains in the printing unit, preferably immobilized to a sample zone. Printing the released detectable label in the course of step c) preferably maintains the orientation of the array of printing units thereby simplifying the correlation of a printed detectable label to the corresponding printing unit and accordingly, the comprised nucleic acid template. This is advantageous for high-throughput applications. The printing device and the substrate can be moved relative to each other thereby allowing to print the released detectable labels from the next sequencing cycle. The printing device may also comprise additionally empty printing units that do not comprise a nucleic acid template. Furthermore, the skilled person may chose not to sequence all nucleic acid templates comprised in the printing units.

A multitude of printing units is considered to be at least 2 printing units. In one embodiment, the printing device comprises at least 10 printing units, at least 100 printing units, at least 200 printing units, at least 500 printing units or at least 1000 printing units. In preferred embodiments the printing device comprises at least 2500 printing units, at least 5000 printing units, at least 10,000 printing units, at least 25,000 printing units, at least 50,000 printing units, at least 75,000 printing units, at least 100,000 printing units, at least 200,000 printing units, at least 300,000 printing units, at least 500,000 printing units, at least 750,000 printing units, at least 1,000,000 printing units, at least 1,500,000 printing units, at least 2,000,000 printing units, at least 3,000,000 printing units, at least 3,500,000 printing units, at least 4,000,000 printing units, at least 4,500,000 printing units or at least 5,000,000 printing units. A printing unit may have e.g. a diameter between 50µm and 5µm, between 40µm and 10µm or between 30µm and 20µm.

In one embodiment the multitude of printing units are arranged along a row, which can be a straight line. The nozzles can be regularly or unregularly spaced and positioned. The term "row" encompasses deviations from a single straight line such that a nozzle and its adjacent predecessor and its adjacent successor are not collinear. The number of printing units along a row can be e.g. more than 50, more than 100, more than 500, more than 1,000, more than 5000, more than 10,000, more than 100,000, more than 1,000,000. In a further arrangement the multitude of printing units are arranged in the style of an array or table, the nozzles of the printing units being arranged e.g. in columns and rows. The number of nozzles in the columns may differ in respective columns. The number of nozzles in the rows may differ in respective rows. In a preferred embodiment the printing device comprises more than 100 printing units, more than 200 printing units, more than 500 printing units or more than 1000 printing units. In preferred embodiments the printing device comprises at least 2500 printing units, at least 5000 printing units, at least 10,000 printing units, at least 25,000 printing units, at least 50,000 printing units, at least 75,000 printing units, at least 100,000 printing units, at least 200,000 printing units, at least 300,000 printing units, at least 500,000 printing units, at least 750,000 printing units, at least 1,000,000 printing units, at least 1,500,000 printing units, at least 2,000,000 printing units, at least 3,000,000 printing units, at least 3,500,000 printing units, at least 4,000,000 printing units, at least 4,500,000 printing units, at least 5,000,000 printing units. The printing units being arranged in at least 100 rows and/or in at least 100 columns, the printing units being arranged in at least 1,000 rows and/or in at least 1,000 columns, the printing units being arranged in at least 10,000 rows and/or in at least 10,000 columns, the printing units being arranged in at least 100,000 rows and/or in at least 100,000 columns or the printing units being arranged in at least 1,000,000 rows and/or in at least 1,000,000 columns. In one embodiment of a printing device with printing units arranged in rows and columns, the majority, preferably all, nozzles are arranged in such a manner that at the majority of cross-sections of rows and columns, preferably at all cross-sections of rows and columns a nozzle is arranged. In a further embodiment, for the majority of rows, optionally for all rows, the nozzles of the respective row are arranged offset from the nozzles at least of one neighbouring row, in embodiments offset from the nozzles of both neighbouring rows. It is preferred to arrange the nozzles of the printing units in a regular pattern. In an alternative embodiment the printing units or nozzles can be arranged in a hexagonal, elliptical or circular alignment.

The arrangement of the printing units or the nozzles can be independent from the pattern printed by a sequence of printing steps. For example, printing units arranged in a row can print a section of an array-like pattern on a substrate in several steps, however, the same printing units arranged in a row can print a section of a circle or ring pattern on a detection substrate. The pattern produced when printing in a sequence of printing steps depends on the movement of the printing units relative to the substrate during each of the printing steps. The detection substrate can have the shape of the printed pattern, however, the shape of the printed pattern can be a section of a circle printed on a rectangular-shaped detection pattern. In an embodiment, a circular-pattern can be printed which can allow for optical readout similar to a readout of a compact disc or blu-ray disc.

In embodiments, the distance between the middle of one nozzle to the middle of the neighbouring nozzle is between 100µm and 1µm, between 90µm and 2µm, between 80µm and 3µm, between 70µm and 5µm, between 60µm and 10 µm, between 55 µm and 15µm, between 50µm and 20µm, between 45µm and 25µm, between 40µm and 30µm. In an embodiment the distance can be e.g. 35µm. For nozzles that have a circular shape, the middle of the nozzle is considered to be the middle of the circle. For nozzles that have non-circular shape, the middle of the nozzle is to be understood as the centre of area of the nozzle.

In a preferred embodiment, the majority of the printing units, preferably all printing units of the printing device, are designed in the same manner.

In one embodiment, each printing unit comprises a reservoir suitable to contain a liquid. The reservoir is in fluid-communication with the nozzle such that a fluid in the reservoir can flow into the nozzle and create a meniscus in the nozzle. Preferably, the reservoir is delimited by sidewalls. As discussed above, the reservoir may comprise a sample zone. In embodiments wherein magnetic particles are used to immobilize the nucleic acid template, the magnetic beads carrying the nucleic acid template (preferably multiple copies thereof) may be immobilized in the reservoir by the aid of a magnetic field. Immobilization occurs preferably in steps a) to c). Details are also described elsewhere herein and it is referred to the respective disclosure.

It is also within the scope of the present invention to use a printing device comprising multiple separate printing heads, wherein each printing head provides a printing unit.

According to one embodiment, at least two of the sidewalls, preferably all of the sidewalls, of the reservoir of the printing units are arranged not in parallel to each other. Preferably the cross-section of the reservoir diminishes towards the nozzle. The reservoir may have the shape of a frustum, preferably the shape of a frustum of a pyramid.

According to one embodiment, each reservoir of a printing unit is separated from the reservoir of a neighbouring printing unit. Accordingly, the reservoirs of each printing unit can be physically separated from each other. The nozzle of a printing unit is likewise separated from the nozzle of a neighbouring printing unit. This allows to prevent contamination between the liquids comprised and processed in the individual reservoirs.

In a further embodiment of a printing device comprising a multitude of printing units, at least some and optionally all of the printing units are provided by sections of a trough, the trough comprising a multiple nozzles, wherein at least one nozzle is provided per printing unit. In this embodiment, the individual printing units are delimited by two facing walls (the walls that form the trough), but are not delimited towards the adjacent printing unit that follows in the direction of extent of the trough. This design allows for a fluid to flow along the trough and hence to flow from printing unit to printing unit. The fluid, as it reaches the respective printing head, filling the nozzle and creating a meniscus in the nozzle. However, the fluid is allowed to leave the printing unit as it flows onwards to the next, neighbouring printing unit. In this embodiment, the printing units are provided in the form of printing zones. The trough may be patterned to simplify the identification of individual printing zones and hence printing units. Details of this embodiment are also described elsewhere herein. One or more or preferably all of these printing units may comprise a sample zone for immobilizing the nucleic acid template as is also shown in the examples. The sample zones may be provided anywhere in the reservoir, e.g. at at least one sidewall of the trough.

According to one embodiment, the template density is at least 10k, at least 50k, at least 75k, at least 100k, at least 150k, at least 200k or at least 250k per 1mm². The template density may be in the range of 200k to 800k per 1mm².

In a preferred embodiment at least a part of the majority of the printing units, preferably all of the printing units, are arranged as part of a printing device carrier. In one embodiment, the printing device carrier is a one-piece element, whereby the printing units form depending on the embodiment one or more sub-pieces of that one-piece carrier element. The carrier may be a piece made from plastic.

In a preferred embodiment, the printing device comprises a multitude of printing units, each printing unit comprising or being connected to a nozzle and being suitable to create a drop out of a meniscus of a liquid arranged in the respective nozzle and have the created drop being urged to travel from the meniscus towards the substrate, if the liquid is placed into the respective nozzle. According to a preferred embodiment a printing device is used which allows to discharge droplets out of a liquid continuum through the multiple nozzles by applying an electric field. The electric field can be used to control the flight trajectory of the droplets that are discharged from the nozzles onto the detection substrate. This allows as explained contactless printing of liquids with an ultra-high resolution and at extremely low liquid volumes. The printing device comprising multiple printing units may thus be an electrohydrodynamic printing device (see above). In a preferred embodiment, the printing device is an electrohydrodynamic printing device of the type described in EP 1 477 230 A1, corresponding WO 2013/000558 or WO2016/12081 to which it is referred for details regarding the printing devices that can be used in conjunction with the method according to the present invention. This printing technology allows ultra-high resolution inkjet printing with nanoscale droplets and moreover discloses according printing devices that comprise densely arranged nozzles.

In a preferred embodiment of the method wherein a printing device comprising multiple printing units is used in order to transfer the released detectable labels in form of droplets in the course of separation step c) to a detection substrate, the transferred droplets are preferably arranged in form of multiple spots on the detection substrate. The arrangement of the spots on the detection unit corresponds to the arrangement of the nozzles of the printing device through which the released detectable label is printed on the detection substrate and the movement of the detection substrate relative to the printing device. Details were described above. In one embodiment the spots are arranged in a row, which can be a straight line. The spots can be regularly or unregularly spaced and positioned. The term "row" encompasses deviations from a single straight line such that a spot and its adjacent predecessor spot and its adjacent successor spot are not collinear. The number of spots along a row can be more than 100, more than 500, more than 1,000, more than 5,000, more than 10,000, more than 25,000, more than 100,000 or more than 1,000,000. In an alternative arrangement the spots are arranged in the style of an array or table, the spots being arranged in columns and rows. In a preferred embodiment the detection substrate comprises more than 100 spots, more than 200 spots, more than 500 spots or more than 1000 spots. In preferred embodiments the detection substrate comprises at least 2500 spots, at least 5000 spots, at least 10,000 spots, at least 25,000 spots, at least 50,000 spots, at least 75,000 spots, at least 100,000 spots, at least 200,000 spots, at least 300,000 spots, at least 500,000 spots, at least 750,000 spots, at least 1,000,000 spots, at least 1,500,000 spots, at least 2,000,000 spots, at least 3,000,000 spots, at least 3,500,000 spots, at least 4,000,000 spots, at least 4,500,000 spots, at least 5,000,000 spots. The spots being arranged in at least 100 rows and/or in at least 100 columns, the spots being arranged in at least 1,000 rows and/or in at least 1,000 columns, the spots being arranged in at least 10,000 rows and/or in at least 10,000 columns, the spots being arranged in at least 100,000 rows and/or in at least 100,000 columns or the spots being arranged in at least 1,000,000 rows and/or in at least 1,000,000 columns. In one embodiment with spots arranged in rows and columns, the majority, preferably all spots are arranged in such a manner that at the majority of cross-sections of rows and columns, preferably at all cross-sections of rows and columns a spot is arranged. In an alternative embodiment, for the majority of rows, preferably for all rows, the spots of the respective row are arranged offset from the spots at least of one neighbouring row, preferably offset from the spots of both neighbouring rows. It is preferred to arrange the spots in a regular pattern. In alternative embodiments, the spots can be arranged in a circular, elliptical, hexagonal or polygonal shape.

The number of spots can be correlated to the number of printing units and the number of sequencing steps.

In one embodiment, the distance between the middle of one spot to the middle of the neighbouring spot for the majority of spots, preferably for all spots is less than 50 micrometers, less than 25 micrometers, less than 15 micrometers or less than 10 micrometers. Preferably, the distance between the middle of one spot to the middle of the neighbouring spot for the majority of spots, preferably for all spots is less than 8 micrometers, less than 7 micrometers, less than 5 micrometers, less than 3 micrometers, less than than 2 micrometers, less than 1 micrometer, less than 0.5 micrometer or less than 0.4 micrometer. In one embodiment, the diameter of a spot, preferably for all spots, is less than 5µm, less than 4µm, less than 3µm, less than 2µm, less than 1µm, less than 0.7µm, less than 0.5µm, less than 0.4µm, less than 0.3µm or less than 0.2µm. For spots that have a circular shape, the middle of the spot is considered to be the middle of the circle. For spots that have non-circular shape, the middle of the spot is to be understood as the centre of area of the spot when the spot is viewed from above.

### Embodiment transfer from a sample substrate to a detection substrate

According to a further embodiment, the method according to the present invention is characterized in that the nucleic acid template is immobilized to a sample substrate. The method may thus comprise a step of immobilizing the nucleic acid template on a definite location on a sample substrate. The sample substrate provides a support structure for the nucleic acid template. The definite location where the nucleic acid template is immobilized is also referred to as sample zone. The nucleic acid template may be attached to the sample zone, examples have been described above. A sample substrate preferably comprises multiple sample zones. The sample zones may have a circular shape, but might also be of rectangular or quadratic shape.

In this embodiment, wherein a sample substrate is used the method may comprise
a) contacting the immobilized nucleic acid template with a hybridizing sequencing reagent comprising a detectable label under suitable conditions to provide a hybrid comprising a detectable label;
b) releasing the detectable label from the hybrid into a liquid medium; and
c) separating released detectable label from the nucleic acid template that is immobilized to the sample substrate by transferring released detectable label comprised in the liquid medium from the sample substrate to a location on a separate detection substrate;
wherein the method further comprises d) detecting the separated detectable label transferred to the detection substrate for determining the sequence.

Suitable sample substrates as well as methods for immobilizing a nucleic acid template to a substrate are well known in the art and therefore, need no detailed description. Suitable substrates were also described above in conjunction with the detection substrate. The same substrates may also be used as sample substrate. The sample substrate may comprise multiple sample zones. The sample zones may be provided in the form of wells. The sample substrate may furthermore have an essentially flat surface. In embodiments, the sample substrate is a microarray comprising multiple sample zones. In embodiments, the sample zones are hydrophilic. The hydrophilic sample zones may be at last partially or completely surrounded by hydrophobic areas. This embodiment is advantageous as it allows to confine the nucleic acid template and the liquids used in the course of the method for processing to the sample zone. This avoids cross-contamination between adjacent sample zones.

A multitude of sample zones is considered to be at least 2 sample zones. Preferably, the sample substrate comprises an array of sample zones. In one embodiment, the sample substrate comprises at least 20 sample zones, at least 50 sample zones, at least 100 sample zones, at least 200 sample zones, at least 500 sample zones or at least 1000 sample zones. In preferred embodiments the sample substrate comprises at least 2500 sample zones, at least 5000 sample zones, at least 10.000 sample zones, at least 25.000 sample zones, at least 50.000 sample zones, at least 75.000 sample zones, at least 100.000 sample zones, at least 250,000 sample zones or at least 500,000 sample zones. With respect to the numbering, essentially the same considerations apply as discussed above in conjunction with the number of printing heads and it is referred to the above discussion.

According to one embodiment, the nucleic acid template is immobilized to a magnetic particle. The magnetic particle(s) comprising a nucleic acid template, preferably comprising multiple copies thereof, may be immobilized via a magnet to the sample zone of the sample substrate.

According to one embodiment, multiple nucleic acid templates are immobilized in different sample zones of the sample substrate thereby forming an array. This advantageously allows the sequencing of multiple different nucleic acid templates in parallel.

According to one embodiment, step c) comprises transferring the released detectable label comprised in the liquid medium in form of droplets from the sample substrate to the definite location on the separate detection substrate. The transfer of released detectable label from a sample zone of the sample substrate to the detection substrate may occur sequentially on a sample zone by sample zone basis. This embodiment is also illustrated in the example section.

According to a preferred embodiment, the transfer from the sample zones of the sample substrate to the detection substrate maintains the orientation of all sample zones.

According to one embodiment the method comprises
- providing a sample substrate comprising different nucleic acid templates immobilized in definite sample zones on the sample substrate thereby providing an array, and
- performing steps a) and b) for different nucleic acid templates immobilized on the sample substrate, and
- wherein for the different nucleic acid templates step c) comprises separating released detectable label from the nucleic acid template that is immobilized in a definite location on the sample substrate by transferring released detectable label comprised in the liquid medium from the sample substrate to a definite location on the detection substrate.

The transfer is performed in a way that allows correlating the transferred detectable labels with the nucleic acid templates. According to one embodiment, the transfer to the detection substrate maintains the array of the different nucleic acid templates as present in the sample zones of the sample substrate. Thus, by transferring aliquots (e.g. small droplets) of the liquid medium comprising the released detectable label that is present on each sample zone where a nucleic acid template is immobilized to the sample substrate it is possible to provide a detectable counterpart of the sample substrate on the detection substrate. The transfer of the aliquot may occur sequentially on a spot by spot basis or in parallel. Importantly, the transfer maintains the array of the sample zones to allow a correlation of the subsequently detected labels to the nucleic acid template from which it originates.

According to one embodiment, at least one reference is additionally provided on the sample substrate. Such reference can be integrated into the array of the sample zones and is also transferred to the detection substrate. This simplifies the correlation of the transferred spots on the detection substrate with the original sample zone array on the sample substrate.

According to one embodiment, the method comprises performing repeated sequencing cycles Xn comprising steps a) to c), wherein the method comprises
- immobilizing the nucleic acid template on a sample zone provided on a sample substrate,
- performing a first sequencing cycle X1 comprising
   a1) contacting the immobilized nucleic acid template with a hybridizing sequencing reagent comprising a detectable label under suitable conditions to provide a hybrid comprising a detectable label;
   b1) releasing the detectable label from the hybrid into a liquid medium applied to the sample zone where the nucleic acid template is immobilized; and
   c1) separating released detectable label from the nucleic acid template that is immobilized to the sample zone by transferring released detectable label comprised in the liquid medium from the sample zone to a location on a separate detection substrate, thereby providing at least one spot comprising the detectable label released during the first sequencing cycle on the detection substrate;
- performing a second sequencing cycle X2 comprising
   a2) contacting the nucleic acid template obtained after the first sequencing cycle which is immobilized to the sample zone with a hybridizing sequencing reagent comprising a detectable label under suitable conditions to provide a hybrid comprising a detectable label;
   b2) releasing the detectable label from the hybrid into a liquid medium applied to the sample zone where the nucleic acid template is immobilized; and
   c2) separating released detectable label from the nucleic acid template that is immobilized to the sample zone by transferring released detectable label comprised in the liquid medium from the sample zone to a location on a separate detection substrate, thereby providing at least one spot comprising the detectable label released during the second sequencing cycle on the detection substrate;
- performing further subsequent sequencing cycles Xn comprising steps an) to cn), wherein step cn) of each further sequencing cycle comprises separating released detectable label from the nucleic acid template that is immobilized to the sample zone by transferring released detectable label comprised in the liquid medium from the sample zone to a location on a separate detection substrate, thereby providing as result of each subsequent sequencing cycle at least one spot comprising the detectable label released during the subsequent sequencing cycle on the detection substrate;
wherein the method further comprises detecting the transferred detectable labels for determining the sequence of the nucleic acid template.

According to one embodiment of said method which comprises performing repeated sequencing cycles Xn comprising steps a) to c), the released detectable labels of at least two or all sequencing cycles are transferred onto the same detection substrate, whereby a detection substrate is provided that comprises spots comprising the detectable label released during two or more or all sequencing cycles of the same nucleic acid template.

According to an alternative embodiment of said method which comprises performing repeated sequencing cycles Xn comprising steps a) to c), the released detectable labels of at least two or all sequencing cycles are transferred onto different detection substrates. According to one embodiment, a provided detection substrate comprises the detectable label released during a single sequencing cycle of a nucleic acid template. An according detection substrate thus provides information on the sequence portion obtained in a single sequencing cycle, e.g. a single base, of the nucleic acid template to be sequenced. The detection of the detectable label and thus the identification of the corresponding sequence portion can be performed independently of further processing steps such as further sequencing cycles. This allows to perform the detection of the detectable label and thus the identification of the corresponding sequence portion in parallel to the further sequencing cycles. E.g. the detection substrate comprising the released detectable labels from a first sequencing cycle can be transferred to an optical device for detection, while the next sequencing cycle is performed in parallel. This allows to shorten the turnaround times. The advantages of such an embodiment are also described elsewhere herein. It is particularly suitable if the sample substrate comprises multiple detection zones, thereby allowing to determine the sequence of multiple nucleic acid templates in parallel.

According to one embodiment, multiple different nucleic acid templates are immobilized in different sample zones of the sample substrate thereby forming an arrangement, e.g. an array. The different sample zones can be arranged along a row, which can be a line, preferably a straight line. The sample zones can be regularly or unregularly spaced and positioned. The term "row" encompasses deviations from a single straight line such that a sample zone and its adjacent predecessor sample zone and its adjacent successor sample zone are not collinear. The number of sample zones along a row can be more than 100, more than 1,000, more than 5,000, more than 10,000, more than 25,000, more than 100,000 or more than 1,000,000. In an alternative arrangement the sample zones are arranged in the style of an array or table, the sample zones being arranged in columns and rows. The number of sample zones in the columns may differ in respective columns. The number of sample zones in the rows may differ in respective rows. The sample zones being arranged in at least 100 rows and/or in at least 100 columns, the sample zones being arranged in at least 1,000 rows and/or in at least 1,000 columns, the sample zones being arranged in at least 10,000 rows and/or in at least 10,000 columns, the sample zones being arranged in at least 100,000 rows and/or in at least 100,000 columns or the sample zones being arranged in at least 1,000,000 rows and/or in at least 1,000,000 columns. In one embodiment with sample zones arranged in rows and columns, the majority, preferably all spots are arranged in such a manner that at the majority of cross-sections of rows and columns, preferably at all cross-sections of rows and columns a sample zone is arranged. In an alternative embodiment, for the majority of rows, preferably for all rows, the sample zones of the respective row are arranged offset from the spots at least of one neighbouring row, preferably offset from the sample zones of both neighbouring rows. It is preferred to arrange the sample zones in a regular pattern, such as in an array. In embodiments, the spots can be arranged in a circular, elliptical, hexagonal or polygonal shape. Steps a) to c) of a sequencing cycle are then performed for these multiple different nucleic acid templates, wherein for multiple different nucleic acid templates step c) comprises separating released detectable label from the nucleic acid template that is immobilized to a sample zone on the sample substrate by transferring released detectable label comprised in a liquid medium from the sample zone of the sample substrate to a definite location on the detection substrate, wherein the transfer maintains the arrangement, e.g. array, of the different nucleic acid templates in the sample zones, whereby a single detection substrate is provided which comprises multiple spots corresponding to the sample zones and comprising the detectable label released during the sequencing cycle of the multiple different nucleic acid templates. For the different nucleic acid templates step c) may comprise separating released detectable label from the nucleic acid template that is immobilized in the sample zone of the sample substrate by transferring released detectable label comprised in the liquid medium from the sample substrate to a definite location on the detection substrate, wherein the transfer maintains the array of the sample zones comprising the different nucleic acid templates.

The diameter of the sample zone can be less than 50 micrometers, less than 25 micrometers, less than 15 micrometers, less than 10 micrometers, less than 8 micrometers, less than 7 micrometers, less than 5 micrometers, less than 3 micrometers, than than 2 micrometers, less than 1 micrometer, less than 0.5 micrometer or less than 0.4 micrometer. Preferably, the distance between the middle of one sample zone to the middle of the neighbouring sample zone for the majority of spots, preferably for all spots is less than 8 micrometers, less than 7 micrometers, less than 5 micrometers, less than 3 micrometers, less than 2 micrometers, less than 1 micrometer, less than 0.5 micrometer or less than 0.4 micrometer.

The density of sample zones of the sample substrate can be between 50,000 and 1,500,000 per 1mm², between 150,000 and 1,000,000 per 1mm² or between 200,000 to 800,000 per 1mm2.

According to one embodiment, the template density is at least 10k, at least 50k, at least 75k, at least 100k, at least 150k, at least 200k or at least 250k per 1mm². The template density may be in the range of 200k to 800k per 1mm². According to one embodiment, the sample substrate has a size of approx. 25-3000 mm², preferably 50-2000mm².

The arrangement of the sample zones can be independent from the pattern transferred by a sequence of transfer steps. For example, sample zones arranged in a single row can transfer a section of an array-like pattern on a substrate in several steps, however, the same sample zones arranged in a single row can transfer a section of a circle or ring pattern on a substrate. The pattern produced when transferring in a sequence of transferring steps depends on the movement of the sample zones relative to the substrate during each of the transfer steps and the arrangement of sample zones of the sample substrate. A transfer pattern can be obtained which allows for a simple detection. For example a circular transfer pattern can be obtained which allows optical readout similar to a compact disc or a blu-ray disc.

According to a preferred embodiment, detecting the transferred detectable labels on the detection substrate for determining the sequence of the nucleic acid template occurs in parallel to performing further processing steps and/or sequencing cycles using the sample substrate.

In a preferred embodiment of the method wherein the sequencing reaction is performed in the sample zone provided on a substrate, one or more reaction fluids required for performing steps a), b) and/or optionally further processing steps, are applied to the sample zones on the substrate by way of printing. According to one embodiment, the method comprises printing one or more liquid reaction mediums and optionally one or more inert liquids (e.g. a wash solution) onto the sample zone(s) comprising the immobilized nucleic acid template. The liquids can be printed onto each sample zone in a way that cross-contamination between the sample zones is prevented. As is explained in detail in the example section, this is advantageous when performing step b), wherein the detectable label is released from the hybrid. During this critical step of releasing the detectable label from the hybrid, a cross-contamination between sample zones comprising different nucleic acid templates must be avoided. Therefore, according to a preferred embodiment, the reagent applied to the sample zone to release the detectable label from the hybrid during step b) (e.g. by cleaving a cleavable linker) is printed onto a sample zone while avoiding contact with other sample zones comprising different nucleic acid templates. As the nucleic acid templates are immobilized to the sample zones, other fluids, such as e.g. the reaction fluid for performing step a) of the method according to the invention and/or wash fluids, can be applied globally to the sample substrate. Such fluids may be e.g. flushed over the sample substrate or the sample substrate may be immersed in such fluids.

Preferably, printing occurs using a printing device comprising multiple nozzles, which may be arranged as array. The array is preferably such that the nozzles apply the discharged liquid precisely to the sample zones so that cross-contaminations between different sample zones is avoided. Preferably, an electrohydrodynamic based printing device as is described in detail above is used for printing the one or more fluids. Moreover, this printing technology allows the nucleic acid templates immobilized on the multiple sample zones to be individually spotted with fluids in several seconds through multiple, e.g. hundreds or thousands, micronozzles in parallel. The nozzles of the printing device are preferably arranged in an arrangement or array that matches the arrangement or array of the sample zones of the sample substrate. This allows precise printing of the fluids to the sample spots. An electrohydrodynamic based printing device may thus be used to apply the reagents required during the sequencing cycles onto the sample zones of the sample substrate. The advantages and suitable printing devices were described in detail above. In a preferred embodiment, the printing device is an electrohydrodynamic printing device of the type described in EP 1 477 230 A1, corresponding WO 2013/000558 or WO2016/12081 to which it is referred for details regarding the printing devices that can be used in conjunction with the method according to the present invention.

As discussed above, printing may be contactless, i.e. the nozzle of the printing device does not contact the sample zone. Printing can occur in a very precise and highly parallel fashion thereby accelerating the process. Sequencing information can be obtained more quickly. Using an according electrohydrodynamic based printing device moreover allows to reduce the costs. As discussed above, the high consumption of reagents in the prior art systems leads to high costs. High consumption is triggered by current design of liquid processing through pumps and tubes which leads to high dead volumes. Using an electrohydrodynamic printing device as described herein that employs e.g. nanodripping avoids these drawbacks, by reducing the amount of reagents used. This allows saving sequencing reagent costs through massively reduced reagent consumption. With respect to the details of the electrohydrodynamic printing device it is referred to the above disclosure.

The electrohydrodynamic based printing technology described herein can moreover be advantageously used to prepare the sample zones on the sample substrate. It can be used e.g. to print immobilization chemistry for immobilizing the nucleic acid template to the sample zone(s). It allows to print dedicated, well defined spots onto the sample substrate (e.g. flow cell) surface at high speed and low cost. It can moreover be used to apply, e.g. seed, the nucleic acid template to the sample zones for immobilization. A micropatterned surface of a sample substrate may comprise functionalized spots as sample zones having a diameter of 10micrometer or less, preferably 5micrometer or less, more preferred 3 micrometer or less or 2 micrometer or less. The spots may have e.g. a diameter of about 1 micrometer with a pitch of 1.5 micrometer. Details are described elsewhere. Using such micropatterned surfaces allows to increase output density. It moreover allows an efficient detection through a well-ordered array of sample zones to which the nucleic acid templates are immobilized. In a preferred embodiment, the printing device is an electrohydrodynamic printing device of the type described in EP 1 477 230 A1, corresponding WO 2013/000558 or WO2016/12081 to which it is referred for details regarding the printing devices that can be used in conjunction with the method according to the present invention.

In a preferred embodiment, the transfer of released detectable label comprised in a liquid medium from the sample substrate to the detection substrate includes the creation of charged droplets of said liquid medium and the transfer of the droplets to a detection substrate, for example by way of letting the droplets drop down or by way of propelling the droplets by use of an electric field. The creation of droplets can be stopped upon transfer of sufficient liquid medium to allow the detection of the released detectable label. Liquid remaining on the sample spots may then be removed and the nucleic acid template can then be further processed (e.g. by performing further sequencing cycles).

The transfer of the released detectable label from the sample substrate to a location on a detection substrate can be performed by way of electrohydrodynamics. Electrohydrodynamics (EHD), also known as electro-fluid-dynamics (EFD) or electrokinetics, are the dynamics of electrically charged fluids and refers also to the motions of ionized particles or molecules and their interactions with electric fields and the surrounding fluid. This principle is used in the printing devices discussed in detail above (see also EP 1 477 230 A1, WO 2013/000558 and WO2016/12081) and may also be used for transferring the released detectable label comprised in a liquid medium in form of small charged droplets from the sample substrate to the detection substrate. For example an electrode can be brought into contact with a sample spot of the sample substrate and a counter-electrode can be brought into contact with the detection substrate. The liquid of the sample spot can be electrically charged and the detection substrate can be electrically charged. The electrical field that is thus created between the sample spot of the first substrate and the second substrate forces small aliquots (e.g. small charged droplets) of the liquid medium comprising released detectable label to transfer from the sample spot on the sample substrate to the detection substrate. Preferably, a sample spot can be provided which is an electrically conductive electrode or can be connected to an electrically conductive electrode. Preferably, the distance between the sample spot on the first substrate and the second substrate is small relative to the strength of the applied electrical field. Preferably, a substantially homogeneous electrical field can be applied.

### Further embodiment of a transfer to a bypass location

In a further embodiment, the transfer of the released detectable label to a bypass-location can be obtained by draining the released detectable label that is comprised in a liquid medium into a tube. This tube is different from a tube or reservoir into which one or more other fluids used in the course or the sequencing process fluid is discharged.

This can be obtained by performing steps a) and b) of the present method in a chamber, for example a microfluid channel or a reservoir, and to provide the chamber with a first closable opening that leads into a first tube and with a second closable opening that leads into a second tube. The openings can be made closable by use of valves. Such an arrangement allows the released detectable label to be transferred in step c) to a bypass-location via the first tube, for example, and to allow other fluids to be discharged from the camber via the second tube, for example. Furthermore, this can be obtained by performing steps a) and b) of the present method in a chamber, for example a microfluid channel or a reservoir and to provide the chamber with an exit tube, wherein a switch or gate is provided in the exit tube that selectively connects the exit tube with a first tube and a second tube. Such an arrangement allows the released detectable label to be transferred to a bypass-location via the first tube, for example, and to allow other fluids used in the course of the sequencing reaction to be discharged from the camber via the second tube, for example.

### Detection

Step d) of the method according to the present invention comprises detecting the separated detectable label. As is well-known in the art detection of the detectable label allows to determine the sequence of the nucleic acid template. Detection devices suitable for various detectable labels commonly used in order to label hybridizing sequencing reagents such as nucleotide analogues and probes are well known in the art and therefore, do not need any detailed description. As discussed above, the label is preferably a fluorescent label such as a fluorescent dye, but also other optical or magnetic detectants can be considered. Details are described elsewhere and are also known in the art.

According to one embodiment, an optical detection device is used in order to detect and identify the label. The detection device may comprise a sensor. Particular preferred, the sensor is an optical sensor. Hence, a contact between the released detectable label and the sensor is not required, however, also possible in embodiments. In one embodiment, the detectable label is directly printed on the sensor. This is e.g. feasible in case of an optical sensor such as a CMOS or CCD camera directly on the individual pixel of the device. In one embodiment, a laser is used to interrogate the detectable label, which is detected by the optical sensor. In a further embodiment, the detection device suitable to detect a characteristic property of the detectable label on the substrate is an optical detection device, preferably a camera, where the optical signal from the label is directed to the sensor by relaying optics such as a lens, or in case of magnification, a microscopic setup, or in case of optical filtering/scanning, a confocal or line-scanning microscopic setup. In another embodiment, a lens-free wide-field optical detection device according to patent US8866063 or similar can be employed.

When the label is a fluorescent label, fluorophore excitation is possible for detection. Fluorophore excitation may be performed by direct illumination. Fluorophore excitation is also possible by evanescent illumination through a (e.g. monochromatic) light source which is coupled into the substrate and guided by total internal reflection (TIR). TIR-guided light can also be filtered by wavelength as needed through the sensor setup.

Detection of the reaction product, especially when it is an optically detectable sequencing byproducts (e.g. fluorophores), can be achieved by a CCD-sensor (charged coupled device) or by a CMOS-sensor (complementary metal-oxide-semiconductor).

As discussed above, the released detectable label comprised in a liquid medium is preferably transferred to a detection substrate where it forms a spot. Detection occurs using the provided detection substrate. Each spot thus contains as result of the transfer detectable sequencing products such as fluorophores. A sensor usually requires a minimum amount of intensity, for example provided by fluorophores under excitation, to safely detect a specific signal. The intensity can be modulated e.g. by the droplet size or volume of liquid medium comprising released detectable label that is e.g. transferred to a detection substrate, where it preferably forms a spot. The quantity of fluorophores can be modulated by number or volume of droplets. This allows to increase the number of fluorophores to the required quantity to allow secure detection.

The minimum size of the spots containing the released detectable through the process are related to the optics utilized for the imaging process. This can be determined by the skilled person and suitable arrangements are described herein. For embodiments lacking a magnification through optics, such as lens-less system, minimum detectable spots are further governed by the utilized sensor resolution. In such a lens-less setup, the resolution of the sensors can be increased by sub-pixel movement of the substrate over the sensor. Therefore, the spot size on the substrate can be smaller than the actual size of one pixel in the sensor. One example of a super resolution microscopy is described in patent US 8866063 B2 in terms of a wide-field imaging process based on sub-pixel sampling of digital holograms.

As discussed herein, the detection of the separated detectable label in the bypass-location, e.g. on a detection substrate, may be performed at least partially at the same time and thus in parallel to performing further processing steps such as e.g. performing intermediate washing steps or performing further sequencing steps.

According to one embodiment, the detection substrate is part of the detection device. It may be part of a sensor for detecting the detectable label. Therefore, the sensor is able to detect the separated detectable label without the necessity to change its position. In one embodiment, the detection device has a lens, the lens being used as detection substrate to carry a drop of a liquid or solid, e.g. dried, medium comprising the separated detectable label.

According to an alternative embodiment, the detection substrate onto which the separated detectable label is transferred is moved to a sensor where detection takes place.

### SYSTEM FOR DETERMINING THE SEQUENCE OF A NUCLEIC ACID TEMPLATE

Also provided is a sequencing system for performing the sequencing method according to the first aspect which has been described in detail above.

The sequencing system may comprise a printing device comprising a reservoir and a nozzle. The reservoir can be provided by or within the nozzle. A nucleic acid template can be immobilized in the reservoir. Details of the printing device and preferred embodiments were described in detail above in conjunction with the method and it is referred to the respective disclosure which also applies here.

The sequencing system may have one or more of the following characteristics:
(i) a nucleic acid template is immobilized in the reservoir;
(ii) the printing device comprised multiple printing units each comprising a nozzle; and/or
(iii) the printing device comprises a heating unit which allows to adjust the temperature in the reservoir.

The device may comprise fluid reservoirs for all required liquids and fluid paths to feed printing units with the desired respective chemistry. It may also comprise fluid paths to provide the DNA sample with chemistry.

According to one embodiment, the sequencing system comprises a detection substrate. As discussed in conjunction with the method, the printing device can print a liquid medium comprised in the reservoir onto the detection device. As discussed above, advantageously, the liquid to be printed may comprise a detectable label released during the sequencing reaction.

In a preferred embodiment the detection substrate can be moved relative to the nozzle of the printing device. In one possible embodiment, the printing device is arranged stationary in the system and the detection substrate is arranged to be moveable relative to the stationary printing device. In one possible embodiment, the detection substrate is arranged stationary in the system and the printing device is arranged to be moveable relative to the stationary substrate. In one possible embodiment, the printing device is arranged moveable in the system and the detection substrate is arranged to be moveable in the system, the relative movement between the printing device and the detection substrate either being obtained by holding the printing device or the detection substrate temporarily relative to the stationary printing device. In a preferred embodiment, where the system comprises a multitude of printing units, a part of the multitude of printing units, preferably all of the printing units are arranged as part of a printing unit carrier, the movement of the (individual) printing units relative to the substrate is achieved by movement of the printing unit carrier. As discussed above, each printing unit comprises a nozzle and may furthermore, comprise a reservoir.

### USE

According to a third aspect, the present invention pertains to the use of a printing device for transferring a detectable label that was released from a nucleic acid hybrid generated in the course of a sequencing reaction to a bypass location, preferably a detection substrate, wherein the transferred detectable label is detected to identify the detectable label. Details with respect to the printing device, the detectable label and the sequencing reaction were described above in conjunction with the method and the respective disclosure also applied here.

According to one embodiment, the printing device comprises a reservoir and a nozzle and wherein a nucleic acid template is provided in the reservoir. As discussed above, the nucleic acid sequencing reaction may be performed in the reservoir of the printing device and detectable label released during the sequencing reaction may be printed onto a detection substrate and wherein the printed detectable label is detected to identify the detectable label. Details were described in conjunction with the method and also apply here.

### FURTHER EMBODIMENTS

Embodiments of the present invention are described again in the following. The present invention in particular also provides for the following items:
1. A method for determining the sequence of a nucleic acid template comprising
   a) contacting the nucleic acid template with a hybridizing sequencing reagent comprising a detectable label under suitable conditions to provide a hybrid comprising a detectable label;
   b) releasing the detectable label from the hybrid; and
   c) separating released detectable label from the nucleic acid template;
   wherein the method further comprises d) detecting separated detectable label for determining the sequence.
2. The method according to item 1, characterized in that separating in step c) involves transferring released detectable label to a bypass-location.
3. The method according to item 1 or 2, characterized in that separating in step c) involves transferring released detectable label to a detection substrate.
4. The method according to item 3, wherein detecting in step d) comprises moving the detection substrate with the transferred detectable label to a detection device to identify the separated detectable label.
5. The method according to item 3 or 4, wherein the detection substrate is part of a detection device.
6. The method according to one or more of items 1 to 5, wherein the sequencing principle is selected from (i) sequencing by synthesis and (ii) sequencing by ligation.
7. The method according to one or more of items 1 to 6, wherein the hybridizing sequencing reagent comprising a detectable label is selected from (i) a nucleotide analogue comprising a nucleobase and a detectable label and (ii) an oligonucleotide probe comprising a detectable label.
8. The method according to one or more of items 1 to 7, wherein the label is an optically detectable label, preferably a fluorescent label, more preferably a fluorescent dye.
9. The method according to one or more of items 1 to 8, wherein the hybridizing sequencing reagent comprises a cleavable linker to which the detectable label is attached.
10. The method according to item 9, wherein the linker is (i) a photocleavable linker or (ii) a chemically cleavable linker.
11. The method according to item 10, wherein (i) if the hybridizing sequencing reagent comprises a photocleavable linker, step b) comprises photocleaving the linker to release the detectable label from the hybrid, or (ii) if the hybridizing sequencing reagent comprises a chemically cleavable linker, step b) comprises adding a cleave reagent to cleave the linker and release the detectable label.
12. The method according to one or more of items 1 to 11, wherein the sequencing principle is sequencing by synthesis by cyclic reversible termination.
13. The method according to one or more of items 1 to 12, wherein the hybridizing sequencing reagent comprising a detectable label is a nucleotide analogue comprising a nucleobase, a detectable label and a reversible terminating group.
14. The method according to item 13, wherein the nucleotide analogue is a 3'O-blocked reversible terminator which comprises as reversible terminating group a removable moiety capping the 3'-OH group of the nucleotide.
15. The method according to item 14, having one or more of the following characteristics:
   (i) the removable moiety capping the 3'-OH group is a cleavable moiety capping the 3'-OH group;
   (ii) the detectable label is attached to the nucleobase;
   (iii) the detectable label is bound to the cleavable capping moiety;
   (iv) the detectable label is attached to a phosphate.
16. The method according to one or more of items 1 to 15, wherein the nucleic acid template has one or more of the following characteristics:
   (i) the nucleic acid template is a DNA template;
   (ii) the nucleic acid template is immobilized in a sample zone at least during step c), preferably during steps a) to c);
   (iii) the nucleic acid template comprises multiple copies of the same strand to be sequenced;
   (iii) the nucleic acid template is provided as DNA nanoball;
   (iv) the nucleic acid template is immobilized on a particle, preferably a magnetic particle;
   (v) the nucleic acid template is a self-priming template.
17. The method according to one or more of items 1 to 16, wherein step a) comprises contacting the nucleic acid template with at least four nucleotide analogues, wherein optionally, the at least four nucleotide analogues have one or more of the following characteristics:
   (i) the first nucleotide analogue is an analogue of A, the second nucleotide analogue is an analogue of G, the third nucleotide analogue is an analogue of T or U and the fourth nucleotide analogue is an analogue of C;
   (ii) at least three or all four nucleotide analogues comprise a detectable label and wherein the four nucleotide analogues are distinguishable;
   (iv) each nucleotide analogue comprises a detectable label;
   (v) each nucleotide analogue comprises a different detectable label.
18. The method according to one or more of items 1 to 17, comprising performing repeated sequencing cycles comprising steps a) to c).
19. The method according to item 18, wherein the method comprises performing repeated sequencing cycles comprising steps a) to c) before detecting the detectable labels separated in the sequencing cycles for determining the sequence of the nucleic acid template.
20. The method according to one or more of items 1 to 19, wherein Xn sequencing cycles comprising steps a) to c) are performed, wherein n is at least 20, at least 25, at least 50, at least 75, at least 100, at least 125 or at least 150.
21. The method according to item 20, wherein detecting the detectable label separated in a sequencing cycle occurs independently and in parallel to further sequencing cycles performed for determining the sequence of the nucleic acid template.
22. The method according to one or more of items 1 to 21, wherein separating in step c) involves transferring released detectable label to a detection substrate and detecting comprises moving the detection substrate with the transferred detectable label to a sensor.
23. The method according to one or more of items 1 to 22, wherein the released detectable label is comprised in a liquid medium and the method comprises printing released detectable label onto a detection substrate.
24. The method according to one or more of items 1 to 23, wherein the released detectable label is comprised in a liquid medium and step c) involves transferring released detectable label to a detection substrate by printing released detectable label through the nozzle of a printing device onto a detection substrate.
25. The method according to one or more of items 1 to 24, having one or more of the following characteristics:
   (i) the method comprises
      - providing a printing device, wherein the nucleic acid template is comprised in the printing device, and
      - performing steps a) and b) in the printing device, and
      - wherein step c) comprises printing released detectable label comprised in a liquid medium onto a detection substrate;
   (ii) the printing device comprises a reservoir and a nozzle, wherein optionally, the reservoir is provided by the nozzle;
   (iii) the method comprises
      - providing a printing device comprising a reservoir and a nozzle, wherein the nucleic acid template is comprised in the reservoir and wherein optionally, the reservoir is provided by the nozzle, and
      - performing steps a) and b) in the reservoir, and
      - wherein step c) comprises transferring released detectable label comprised in a liquid medium through the nozzle onto a detection substrate.
26. The method according to one or more of items 23 to 25, wherein released detectable label comprised in a liquid medium is printed in a definite location on the detection substrate.
27. The method according to one or more of items 1 to 26, comprising performing repeated sequencing cycles Xn comprising steps a) to c), wherein the method comprises
   - providing a printing device comprising a nozzle, wherein the nucleic acid template is comprised in the printing device, preferably in a reservoir,
   - performing a first sequencing cycle X1 comprising
      a1) contacting within the printing device the nucleic acid template with a sequencing reagent comprising a detectable label under suitable conditions to provide a hybrid comprising a detectable label;
      b1) releasing the detectable label from the hybrid into a liquid medium comprised in the printing device; and
      c1) separating released detectable label from the nucleic acid template that is comprised in the printing device wherein separating comprises transferring released detectable label comprised in the liquid medium through the nozzle onto a detection substrate, thereby providing at least one spot comprising the detectable label released during the first sequencing cycle on the detection substrate;
   - performing a second sequencing cycle X2 comprising
      a2) contacting within the printing device the nucleic acid template obtained after the first sequencing cycle with a sequencing reagent comprising a detectable label under suitable conditions to provide a hybrid comprising a detectable label;
      b2) releasing the detectable label from the hybrid into a liquid medium comprised in the printing device; and
      c2) separating released detectable label from the nucleic acid template that is comprised in the printing device wherein separating comprises transferring released detectable label comprised in the liquid medium through the nozzle onto a detection substrate, thereby providing at least one spot comprising the detectable label released during the second sequencing cycle on the detection substrate;
   - performing further subsequent sequencing cycles Xn comprising steps an) to cn), wherein step cn) of each further sequencing cycle comprises separating released detectable label from the nucleic acid template that is comprised in the printing device wherein separating comprises transferring released detectable label comprised in a liquid medium through the nozzle onto a detection substrate, thereby providing as result of each subsequent sequencing cycle at least one transferred spot comprising the detectable label released during each subsequent sequencing cycle on a detection substrate;
   wherein the method further comprises detecting the spotted detectable labels for determining the sequence of the nucleic acid template.
28. The method according to item 27, wherein the released detectable labels of at least two or all sequencing cycles are transferred onto the same detection substrate, whereby a detection substrate is provided that comprises spots comprising the detectable label released during two or more, or all sequencing cycles.
29. The method according to item 27, wherein the released detectable labels of at least two or all sequencing cycles are transferred onto different detection substrates.
30. The method according to one or more of items 23 to 29, having one or more of the following characteristics:
   (i) released detectable label comprised in a liquid medium is discharged as one or more droplets from the nozzle onto the detection substrate to form a spot in a definite location on the detection substrate;
   (ii) the nozzle of the printing device does not contact the detection substrate during the printing process;
   (iii) the printing device has a nozzle that is suitable to create a drop out of a meniscus of a liquid arranged in the nozzle, wherein the created drop is urged to travel from the meniscus towards the substrate, if liquid is placed into the nozzle;
   (iv) the printing device has an electrode that is suitable to come into contact with a liquid, if the liquid is placed into the nozzle and has a counter-electrode; wherein optionally/preferably the counter-electrode is in contact with the detection substrate;
   (v) the printing device allows to discharge nanodroplets out of a liquid continuum by applying an electric field; and/or
   (vi) the printing device is an electrohydrodynamic printing device.
31. The method according to one or more of items 23 to 30, characterized in that the method comprises using a printing device comprising a multitude of nozzles.
32. The method according to one or more of items 23 to 31, characterized in that the method is performed using a printing device comprising a multitude of printing units wherein each printing unit comprises or is connected to a nozzle.
33. The method according to one or more of items 23 to 32, wherein the printing device comprises different nucleic acid templates in different printing units, wherein each printing unit comprises or is connected to a nozzle.
34. The method according to one or more of items 23 to 33, characterized in that the method is performed using a printing device comprising an arrangement or array of printing units, each printing unit comprising or being connected to a nozzle and each printing unit comprising a nucleic acid template to be sequenced, wherein steps a) to c) are performed in parallel in each printing unit comprising a nucleic acid template and wherein step c) comprises transferring released detectable label comprised in a liquid medium through each nozzle onto a detection substrate.
35. The method according to one or more of items 1 to 22, characterized in that the nucleic acid template is immobilized to a sample substrate.
36. The method according to item 35, comprising
   a) contacting the immobilized nucleic acid template with a hybridizing sequencing reagent comprising a detectable label under suitable conditions to provide a hybrid comprising a detectable label;
   b) releasing the detectable label from the hybrid into a liquid medium; and
   c) separating released detectable label from the nucleic acid template that is immobilized to the sample substrate by transferring released detectable label comprised in the liquid medium from the sample substrate to a location on a separate detection substrate;
   wherein the method further comprises detecting the separated detectable label transferred to the detection substrate for determining the sequence.
37. The method according to item 35 or 36, wherein the sample substrate comprises a sample zone and the nucleic acid template is immobilized to the sample zone.
38. The method according to one or more of items 35 to 37, wherein multiple nucleic acid templates are immobilized in different sample zones of the sample substrate thereby forming an arrangement or array.
39. The method according to one or more of items 35 to 38, wherein step c) comprises transferring the released detectable label comprised in the liquid medium in form of droplets from the sample substrate to the definite location on the separate detection substrate.
40. The method according to one or more of items 35 to 39, wherein the transfer to the detection substrate maintains the arrangement, e.g. array, of all sample zones.
41. The method according to one or more of items 35 to 40, comprising
   - providing a sample substrate comprising different nucleic acid templates immobilized in definite sample zones on the sample substrate thereby providing a sample zone arrangement or array, and
   - performing steps a) and b) for different nucleic acid templates immobilized on the sample substrate, and
   - wherein for the different nucleic acid templates step c) comprises separating released detectable label from the nucleic acid template that is immobilized to a sample zone by transferring released detectable label comprised in the liquid medium from the sample zone to a definite location on a detection substrate.
42. The method according to one or more of items 35 to 41, wherein the transfer to the detection substrate maintains the arrangement or array of the different nucleic acid templates as present in the sample zones of the sample substrate.
43. The method according to one or more of items 35 to 42, comprising performing repeated sequencing cycles Xn comprising steps a) to c), wherein the method comprises
   - immobilizing the nucleic acid template to a sample zone provided on a sample substrate,
   - performing a first sequencing cycle X1 comprising
      a1) contacting the immobilized nucleic acid template with a hybridizing sequencing reagent comprising a detectable label under suitable conditions to provide a hybrid comprising a detectable label;
      b1) releasing the detectable label from the hybrid into a liquid medium applied to the sample zone where the nucleic acid template is immobilized; and
      c1) separating released detectable label from the nucleic acid template that is immobilized to the sample zone by transferring released detectable label comprised in the liquid medium from the sample zone to a location on a separate detection substrate, thereby providing at least one spot comprising the detectable label released during the first sequencing cycle on the detection substrate;
   - performing a second sequencing cycle X2 comprising
      a2) contacting the nucleic acid template obtained after the first sequencing cycle which is immobilized to the sample zone with a hybridizing sequencing reagent comprising a detectable label under suitable conditions to provide a hybrid comprising a detectable label;
      b2) releasing the detectable label from the hybrid into a liquid medium applied to the sample zone where the nucleic acid template is immobilized; and
      c2) separating released detectable label from the nucleic acid template that is immobilized to the sample zone by transferring released detectable label comprised in the liquid medium from the sample zone to a location on a separate detection substrate, thereby providing at least one spot comprising the detectable label released during the second sequencing cycle on the detection substrate;
   - performing further subsequent sequencing cycles Xn comprising steps an) to cn), wherein step cn) of each further sequencing cycle comprises separating released detectable label from the nucleic acid template that is immobilized to the sample zone by transferring released detectable label comprised in the liquid medium from the sample zone to a location on a separate detection substrate, thereby providing as result of each subsequent sequencing cycle at least one spot comprising the detectable label released during the subsequent sequencing cycle on the detection substrate;
   wherein the method further comprises detecting the transferred detectable labels for determining the sequence of the nucleic acid template.
44. The method according to item 43, wherein the released detectable labels of at least two or all sequencing cycles are transferred onto the same detection substrate, whereby a detection substrate is provided that comprises spots comprising the detectable label released during two or more or all sequencing cycles of the same nucleic acid template.
45. The method according to item 43, wherein the released detectable labels of at least two or all sequencing cycles are transferred onto different detection substrates.
46. The method according to one or more of items 35 to 45, wherein multiple different nucleic acid templates are immobilized in different sample zones of the sample substrate, whereby a sample zone arrangement or array comprising immobilized nucleic acid templates is provided.
47. The method according to item 46, wherein steps a) to c) of a sequencing cycle are performed for these multiple different nucleic acid templates, wherein for multiple different nucleic acid templates step c) comprises separating released detectable label from the nucleic acid template that is immobilized to a sample zone on the sample substrate by transferring released detectable label comprised in a liquid medium from the sample zone of the sample substrate to a definite location on the detection substrate, wherein the transfer maintains the array of the different nucleic acid templates in the sample zones, whereby a single detection substrate is provided which comprises multiple spots corresponding to the sample zones and comprising the detectable label released during the sequencing cycle of the multiple different nucleic acid templates.
48. The method according to item 47, wherein for the different nucleic acid templates step c) comprises separating released detectable label from the nucleic acid template that is immobilized in the sample zone of the sample substrate by transferring released detectable label comprised in the liquid medium from the sample substrate to a definite location on the detection substrate, wherein the transfer maintains the array of the sample zones comprising the different nucleic acid templates.
49. The method according to item 35 to 48, wherein detecting the transferred detectable labels on the detection substrate for determining the sequence of the nucleic acid template occurs in parallel to performing further sequencing cycles using the sample substrate.
50. The method according to one or more of items 35 to 49, comprising printing one or more liquid mediums, preferably liquid reaction mediums, onto the sample zone(s) comprising the immobilized nucleic acid template.
51. The method according to one or more of items 35 to 50, wherein one or more liquid mediums, preferably liquid reaction mediums, are printed onto each sample zone in a way that cross-contamination between the sample zones is prevented.
52. The method according to any one of items 49 to 51, wherein printing occurs using a printing device comprising multiple nozzles, wherein optionally, the nozzles are arranged as array.
53. The method according to one or more of items 50 to 52, wherein the printing device has one or more of the following characteristics:
   (i) the nozzle of the printing device does not contact the sample zone during the printing process;
   (ii) the printing device has a nozzle that is suitable to create a drop out of a meniscus of a liquid arranged in the nozzle, wherein the created drop is urged to travel from the meniscus towards the sample substrate, if liquid is placed into the nozzle;
   (iii) the printing device has an electrode that is suitable to come into contact with a liquid, if the liquid is placed into the nozzle and has a counter-electrode; wherein optionally/preferably the counter-electrode is in contact with the sample substrate;
   (iv) the printing device allows to discharge nanodroplets out of a liquid continuum by applying an electric field;
   (v) the printing device is an electrohydrodynamic printing device; and/or
   (vi) the printing device comprises multiple nozzles that are arranged to allow printing of a liquid precisely onto sample zones provided on a corresponding sample substrate.
54. A sequencing system for performing the sequencing method according to any one of items 1 to 53.
55. The sequencing system according to item 54, comprising a printing device.
56. The sequencing system according to item 55, having one or more of the following characteristics:
   (i) the printing device comprises a reservoir and a nozzle, wherein a nucleic acid template can be immobilized in the reservoir;
   (ii) a nucleic acid template is immobilized in the printing device, preferably in reservoir comprising or being connected to a nozzle;
   (iii) the printing device comprised multiple printing units each comprising a nozzle, wherein optionally, different nucleic acid templates are immobilized in different printing units; and/or
   (iv) the printing device comprises a heating and/or cooling unit which allows to adjust the temperature in the reservoir.
57. The sequencing system according to any one of items 54 to 56, wherein the sequencing system has one or more of the following features:
   (i) it comprises a detection substrate, wherein optionally, the detection substrate can be moved relative to the nozzle;
   (ii) it comprises or is connected to a detection system;
   (iii) it comprises a first printing device, comprising at least one sample zone for immobilizing a nucleic acid template, and optionally comprises a second printing device adapted to supply fluids to the first printing device.
58. Use of a printing device for transferring a detectable label that was released from a nucleic acid hybrid generated in the course of a sequencing reaction to a bypass location, preferably a detection substrate, wherein the transferred detectable label is detected to identify the detectable label.
59. The use according to item 58, wherein the printing device comprises a nucleic acid template.
60. The use according to item 59, wherein the nucleic acid sequencing reaction is performed in the printing device and wherein detectable label released during the sequencing reaction is printed onto a detection substrate and wherein the printed detectable label is detected to identify the detectable label.
61. The use according to item 58 or 59, wherein the printing device comprises a reservoir and wherein the nucleotide template is immobilized in the reservoir, and wherein preferably, the sequencing reaction is performed in the reservoir and wherein detectable label released during the sequencing reaction is printed through a nozzle onto a discrete location on a detection substrate.

This invention is not limited by the exemplary methods, uses, systems and materials disclosed herein, and any methods, uses, systems and materials similar or equivalent to those described herein can be used in the practice or testing of embodiments of this invention. Numeric ranges are inclusive of the numbers defining the range.

The headings provided herein are not limitations of the various aspects or embodiments of this invention which can be read by reference to the specification as a whole.

As used in the subject specification, the singular forms "a", "an" and "the" include plural aspects unless the context clearly dictates otherwise. Thus, for example, reference to "a nozzle" includes a single nozzle, as well as two or more nozzles. Reference to "the disclosure" and "the invention" and the like includes single or multiple aspects taught herein; and so forth. Aspects taught herein are encompassed by the term "invention".

It is preferred to select and combine preferred embodiments described herein and the specific subject-matter arising from a respective combination of preferred embodiments also belongs to the present disclosure.

### BRIEF DESCRIPTION OF THE FIGURES

- Fig. 1: shows a schematic view of an exemplary embodiment of the sequencing system and the sequencing method wherein a printing device is used
- Fig. 2: shows a schematic view of an exemplary embodiment of the sequencing system and the sequencing method wherein a printing device comprising a trough shaped reservoir is used
- Fig. 3: shows a schematic view of a sample substrate that can form part of the system according to the invention and can be used in the method according to the invention
- Fig. 4: shows a schematic view of the sample substrate of Fig. 3 with DNA templates seeded and immobilized to the sample zones
- Fig. 5: shows a schematic view of the sample substrate of Fig. 4 wherein a fluid comprising the reactive chemistry for performing step a) of the method of the invention is globally applied to the sample substrate
- Fig. 6 a) - c): provides a simplified overview of core steps that can be performed in a sequencing cycle
- Fig. 7: shows a schematic view of the sample substrate of Fig. 5 with a wash fluid applied to the sample substrate to wash-off reactive chemistry
- Fig. 8: shows a schematic view of the sample substrate of Fig. 7 after the wash fluid was removed
- Fig. 9: shows a schematic view of the sample substrate of Fig. 8 and a printing device that applies a reaction fluid to the sample zones of the sample substrate
- Fig. 10: shows a schematic view of the sample substrate of Fig. 9 with the individual fluorescent dyes released into the liquid medium located in each sample zone of the sample substrate
- Fig. 11: shows a schematic view of the sample substrate of Fig. 10 and how small droplets of the liquid medium comprising the fluorescent dye are transferred from the sample zones to the bypass-locations provided on the separate detection substrate
- Fig. 12: provides a simplified overview of core steps that can be performed in a sequencing cycle
- Fig. 13: shows a schematic view of a further exemplary embodiment of the system
- Fig. 14: shows a schematic view of yet a further exemplary embodiment of the system
- Fig. 15: shows different options for modified nucleotide analogues.

The invention is described below by way of embodiments by referring to the figures which are not intended to be limiting to the scope of the invention.

Fig. 1 shows a schematic view of an exemplary embodiment of the sequencing system and the sequencing method according to the invention wherein a printing device 1 is used in order to print detectable label released in step b) of the method according to the invention onto a detection substrate 5 as an embodiment of a bypass location. The printing device 1 comprises a reservoir 2 which comprises in the shown embodiment a liquid medium. The reservoir 2 comprises immobilized to its sidewall a nucleic acid template 3. In the shown example, the nucleic acids template 3 is provided in the form of a DNA nanoball (rolony). The area where the nucleic acid template 3 is immobilized to the surface of the reservoir is also referred to as sample zone. Figure 1 shows the sequencing system respectively the sequencing method at a stage, wherein steps a) and b) of the method according the invention have already been performed in the reservoir 2 of the printing device 1 and the detectable label has been released from the hybrid into the liquid medium that is present in the reservoir 2 of the printing device 1. The detectable label floats freely in the liquid medium. Thus, when transferring an aliquot of the liquid medium, released detectable label is likewise transferred. The printing device 1 furthermore comprises a nozzle 4 which is connected to the reservoir 2 and provides an exit out of the reservoir 2 for the liquid that is comprised therein.

The printing device 1 is used to print and thereby transfer released detectable label comprised in a liquid medium to a detection substrate 5. The transferred released detectable label is thereby separated from the DNA template 3 which remains immobilized to the sample zone in the reservoir 2. As is shown, printing can be performed contact less so that the nozzle 4 does not contact the detection substrate 5. The detection substrate 5 is a substrate in form of a plate. The plate is in this shown embodiment movable between two positions. In the first position 6a, the detection substrate 5 is arranged under the printing device 1 where the detection substrate 5 can receive released detectable label comprised in a liquid medium. The liquid medium is transferred from the reservoir 2 through the nozzle 4 to the detection substrate 5. The printing device 1 transfers the released detectable label in this shown preferred embodiment in form of nanosized small droplets 8 of liquid medium which comprises the released detectable label. As is described in the general description, this can be achieved by using a printing device applying an electric field. The printing device is preferably an electrohydrodynamic based printing device. The means for generating and applying the electrical field (e.g. cathode and anode) may also be part of the printing device 1 and/or the sequencing system. By applying an electric field, the shape, size and flight trajectory of the charged droplets 8 can be determined. This allows to adjust the volume of liquid medium that is transferred to the detection substrate 5 according to the demands. After leaving the reservoir 2 of the printing device 1 through the nozzle 4, the small charged droplets 8 travel through the space between the nozzle 4 and the detection substrate 5, which is in the first position 6a. According to a preferred embodiment, several droplets 8 are transferred to the detection substrate 5 in a defined location, here in form of a discrete spot 9. When the spot 9 of transferred detectable label reaches the required size/volume, the transfer of droplets 8 can be terminated and the detection substrate 5 can be moved in the second position 6b for detecting the printed released detectable labels. In the second position 6b, the detection substrate 5 is moved away from the printing device 1 to a detection unit, here an optical detection unit that is capable of detecting the fluorescence of the detectable label. In this shown embodiment, the detection substrate 5 is arranged above a sensor 7 of a detection unit. The sensor 7 analyzes released detectable label that has been deposited as spot 9 on the detection substrate 5. For example, where the label is a fluorescent label, fluorophore excitation is possible for detection. Fluorophore excitation may be performed by direct illumination. Details are known in the art. Advantageously, the detection of a fluorescent label is also possible, if the spot 9 dries during processing/handling. The transferred detectable label, e.g. a fluorescent label, can nevertheless be determined, thereby allowing to securely identify the sequence of the nucleic acid template. This simplifies the processing. Decoupling the sequencing reaction from the detection of the released detectable label as is taught by the present invention has several important advantages as has been described above.

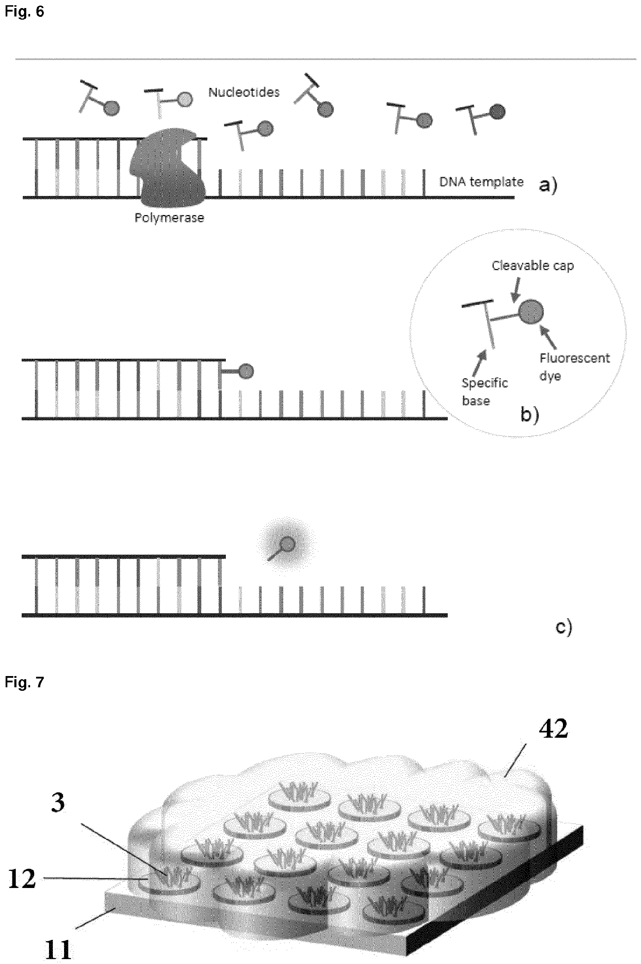

While the detection substrate 5 is being moved in the second position 6b and/or while the sensor 7 analyses the spot 9, the remaining liquid medium in the reservoir 2 of the printing device can be drained out of the reservoir 2 into a drainage or waste container (not shown). This may occur through the nozzle 4. An according waste/drainage container may be located below the nozzle 4. The DNA template 3 in the reservoir 2 may then be optionally further processed, e.g. washed, by introducing one or more processing fluids into the reservoir and subsequent removal (e.g. through the nozzle 4). The next sequencing cycle may then begin by introducing a hybridizing sequencing reagent comprising a detectable label into the reservoir 2 of the printing device 1. The individual steps of an exemplary sequencing cycle are explained in conjunction with Fig. 6.

Fig. 2 shows an excerpt of a printing device with a trough shaped reservoir 2 comprising multiple nozzles 4a to 4c. In the shown excerpt of the reservoir 2, three sample zones are provided, wherein a different nucleic acid template (rolony) 3a-3c is immobilized to each sample zone. The shown excerpt of the trough shaped reservoir provides three printing zones 10a to 10c, wherein each printing zone comprises a nozzle 4a to 4c. The different DNA templates 3a-3c that are immobilized to their sample zones of the reservoir 2 can be contacted sequentially with different fluids that are used in the course of the sequencing reaction by way of letting the fluids flow along the trough. The fluids can be moved across the printing zones 10a to 10c, thereby allowing to contact the different DNA templates 3a-3c sequentially with the different fluids used during sequencing. The flow of the fluids along the trough and hence the printing zones 10a to 10c can be controlled. To avoid contaminations of different reaction fluids used for sequencing during the flow, a reaction fluid can be followed by an inert fluid (e.g. wash buffer or oil). This allows to control the position and distribution of the reaction fluids along the trough. Hence, a sequence of reaction fluids can be provided, each reaction fluid being separated from the neighbouring reaction fluid by an inert liquid functioning as liquid plug. The sequence of different reaction fluid, liquid plug and reaction fluid is made to flow along the trough and hence through the reservoir 2 and in doing is brought into contact with the DNA templates 3a to 3c sequentially. Details are also described elsewhere herein.

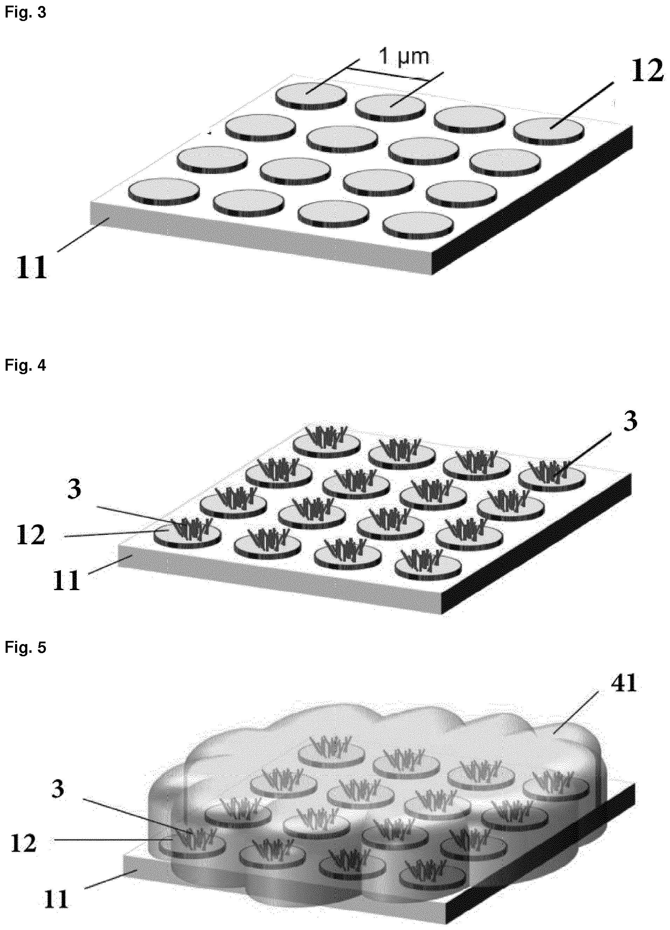

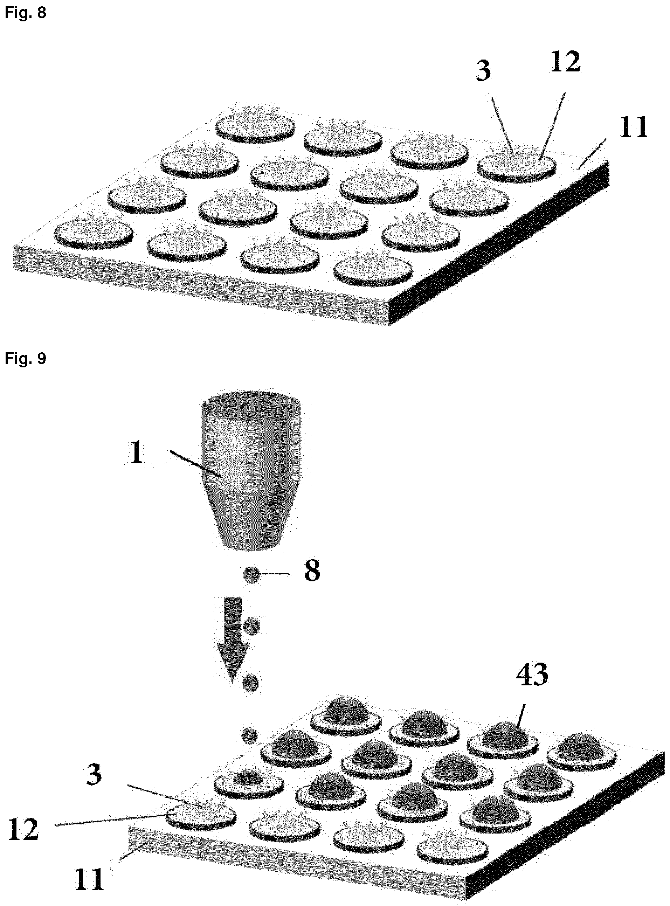

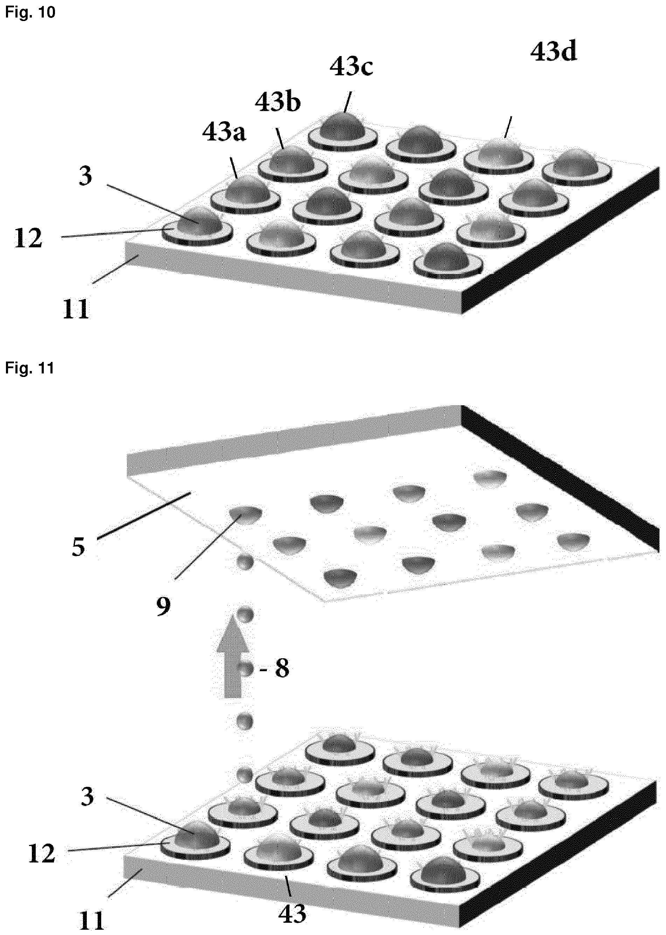

Figs. 3 to 11 illustrate how the method according to the invention can be performed using a sample substrate comprising sample zones to which different nucleic acid templates are immobilized. In short, core steps are
- the preparation of sample zones on a substrate to provide a sample substrate (Fig. 3),
- the immobilization of different nucleic acid templates to the sample zones (Fig. 4),
- the application of a reaction fluid comprising the reactive chemistry required for performing step a) of the method (Fig. 5),
- the removal of the reaction fluid by washing (Figs. 7 and 8),
- the application of a reaction fluid for performing step b) of the method to release the detectable label during an incubation step (Figs. 9 and 10), and
- the transfer of liquid medium comprising released detectable label to a detection substrate (Fig. 11).

Any liquid medium comprising released detectable label that remains on the sample zones after the transfer of released detectable label to the detection substrate may then be removed by a wash step (not shown) and a new sequencing cycle can begin by applying again a reaction fluid comprising the reactive chemistry required for performing step a) of the method. The detection of the detectable labels transferred to the detection substrate can advantageously occur independently and in parallel to the further sequencing steps. This shortens turnaround times. The different steps are now explained in more detail by referring to the Figs.

Fig. 3 shows a sample substrate 11 with multiple sample zones 12. The substrate can be made e.g. from glass or silica. The sample zones 12 are arranged in a regular array pattern with an approx. 1 micrometer distance between the center of each sample zone 12. The sample zones 12 may be hydrophilic and may be surrounded by hydrophobic areas. The sample zones 12 may be patterned, e.g. by interferometric laser ablation. The sample zones 12 comprise a surface modification for immobilizing the DNA templates, preferably by covalent attachment. Suitable immobilization chemistries are known to the skilled person. In a large scale operation, a sample substrate 11 may comprise a sample zone density of as many as 400.000 sample zones per squaremillimeter.

Fig. 4 shows a schematic view of the sample substrate of Fig. 3, wherein different DNA templates 3 are immobilized to the sample zones 12. The DNA templates 3 are provided onto the sample zones 12 and immobilized. In one embodiment, the DNA template 3 is a DNA nanoball (rolony). A rolony preferably comprises at least 100 copies/concatemers of a specific monoclonal DNA fragment to provide sufficient copies of the nucleic acid template to be sequenced. The resulting sample substrate 11 is thereby loaded with different DNA templates 3 and is ready for sequencing.

Fig. 5 shows a schematic view of the sample substrate of Fig. 4 wherein a reaction fluid 41 comprising the reactive chemistry for performing step a) of the method according to the invention is applied to the sample zones 12 of the sample substrate 11. As the different DNA templates 3 are immobilized to their sample zones, the reaction fluid 41 may be globally applied to the sample substrate 11 and the DNA templates 3 provided on the sample zones 12 of the sample substrate 11 as is illustrated in Fig. 5. E.g. the sample substrate 11 may be flushed with the reaction fluid 41 or the sample substrate 11 may be immersed in the reaction fluid 41. The composition of the reaction fluid will depend *inter alia* on the sequencing principle used (e.g. SBL or SBS). The reaction fluid 41 comprises e.g. the hybridizing sequencing reagent comprising a detectable label (e.g. a labelled probe for SBL or labelled nucleotides for SBS), the enzyme required to incorporate hybridizing sequencing reagent comprising a detectable label (e.g. a ligase for SBL and a polymerase for SBS) and one or more primers to initiate the reaction in cases where the DNA template is not a self-priming template. Suitable reaction fluids and conditions are known to the skilled person.

The sequencing reaction is illustrated in further detail in Fig. 6, here based on an embodiment wherein the sequencing reaction is based on sequencing by synthesis by cyclic reversible termination. The reaction fluid 41 comprising the reactive chemistry for performing step a) of the method may comprise a DNA polymerase and single A-, G-, T- and C-nucleotides, which are each modified by the attachment of a detectable label (in the shown embodiment a fluorescent dye) and a capping moiety. Accordingly modified nucleotides are also referred to herein as nucleotide analogues and are preferably used as hybridizing sequencing reagent comprising a detectable label in the context of the invention. As is shown in the enlargement in Fig. 6b), the nucleotide analogues that may be used as hybridizing sequencing reagent comprising a detectable label comprise a specific nucleobase (A, G, T or C), a fluorescent dye and a removable capping moiety which blocks the 3'OH group of the nucleotide. The cap prevents extension with another nucleotide in its proximity on the DNA template. Each nucleotide is labelled with a different fluorescent dye, which either emits blue, yellow, green or red light. The fluorescent dyes are thus specific for the nucleotides that they are attached to. This allows to distinguish the different nucleotides based on the detectable label. The detectable label may be directly attached to the nucleotide as it is known in the prior art. In the embodiment shown in Fig. 6, the detectable label is attached via the cleavable capping moiety to the nucleotide. In step a) of the method according to the invention, a specific nucleotide hybridizes to a complementary base of the DNA template thereby providing a hybrid comprising a detectable label (see Fig. 6a). The reaction is catalyzed by a polymerase and a free 3'OH group for incorporating the labelled complementary nucleotide may be provided e.g. by a suitable primer that hybridizes to a sequencing adapter provided in the DNA template, or the DNA template itself if a self-priming template is used. After incorporation of a labelled nucleotide into a hybrid, the capping moiety prevents the incorporation of a further nucleotide (see Fig. 6b). The reaction fluid 41 may then be removed, e.g. by performing a wash step (see also Fig. 7 below) to remove unincorporated labelled nucleotides. In step b) of the method according to the present invention, the detectable label is released from the formed hybrid. In the shown embodiment, this is achieved by cleaving the cleavable capping moiety. This not only removes the capping moiety (thereby restoring the 3'-OH group of the incorporated nucleotide), but at the same time releases the detectable label from the hybrid, because the detectable label is attached to the cleavable cap. The cleaved cap and the detectable label (fluorophore) are floating in the liquid medium used for cleaving the cleavable capping moiety. This is illustrated in Fig. 6c (see also Figs. 9 and 10 below). In step c) of the method of the invention, liquid medium comprising the released detectable label is then separated from the nucleic acid template, e.g. by transferring an aliquot to a detection substrate (see Fig. 11 below) and the separated detectable label is then detected, e.g. using fluorescence detection in case a fluorescent dye is used as detectable label. The fluorescence signals of the cleaved chemistry may thus be detected independently of the presence of the DNA template.

As discussed, Fig. 5 illustrates the application of the reaction fluid 41 comprising the reactive chemistry for performing step a) of the method according to the invention to the sample zones 12 of the sample substrate 11. As a result of the reaction, a hybrid of the nucleic acid template is provided which comprises the fluorescent dye of the incorporated nucleotide. The reaction fluid 41 and all nucleotides that were not incorporated are washed away with a wash fluid. Fig. 7 shows a schematic view of the sample substrate 11 of Fig. 5 with a wash fluid 42 applied to the sample substrate 11 in order to wash-off the reactive chemistry (in particular unincorporated labelled nucleotides) used in step a) of the method of the invention. As the nucleic acid templates 3 are immobilized to the sample zones 12, the wash fluid 42 can be globally applied to the sample substrate 11. The hybrid comprising the incorporated detectable label provided in step a) of the method of the invention will remain attached to the sample zone due to the immobilization of the nucleic acid template 3 to the sample zone 12. The wash fluid 42 is then removed, thereby providing the washed hybrids of the nucleic acid templates 3 which comprise the detectable label (fluorescent dye) incorporated in step a) of the method of the invention (see Fig. 8).

Step b) of the method of the invention comprises releasing the detectable label (e.g. the fluorescent dye) from the hybrid that was provided in step a). As discussed in conjunction with Fig. 6, this can be advantageously achieved by applying a reaction fluid comprising reagents suitable to cleave of the detectable label from the hybrid. This is further illustrated in Figs. 9 and 10. Fig. 9 shows a schematic view of the sample substrate 11 of Fig. 8 and a printing device 1 that applies a reaction fluid 43 suitable to cleave-off the detectable label to the sample zones 12 of the sample substrate 11. The cleave chemistry 43 may at the same time remove the capping moiety. It is however also possible to release (e.g. cleave-off) the detectable label and to remove the capping moiety in separate processing steps as it is also known in the art. The cleave chemistry 43 is printed in form of small droplets 8 onto each sample zone 12 in a way that prevents cross-contamination between different sample zones 12 which comprise different nucleic acid templates 3. The cleave chemistry 43 may form an individual droplet on each sample zone 12 as is illustrated in Fig. 9. This prevents an intermixing of released detectable labels from different sample zones comprising different DNA templates 3. Printing can also occur with multiple printing units (only one shown in Fig. 11 for reasons of clarity) and hence in a highly parallel fashion to accelerate this method step. The cleave chemistry releases at least the detectable label from the hybrid, which as a result floats in the droplet 43 that was applied to the sample zone 12. As discussed above, it is advantageous to attach the fluorescent dye to the cleavable cap that blocks the 3'-OH group of the nucleotide. The sample substrate 11 may be incubated during step b) as it is shown in Fig. 10. As is shown in Fig. 10, as a result of step b), the released detectable label is comprised in the liquid medium 43 that was used to release the detectable label from the hybrid. In the shown embodiment, different DNA templates 3 are immobilized to different sample zones 12. Thus, different nucleotides were incorporated during step a) (depending on the sequence of the nucleic acid template) and accordingly, different fluorescent dyes are released into the liquid medium droplets 43 present on the individual sample zones (see 43a-43d), depending on the labelled nucleotide incorporated in step a). It is therefore important to prevent cross-contamination between the droplets 43 present on the different sample zones 12.

Fig. 11 shows a schematic view of the sample substrate of Fig. 10 and how the released fluorescent dyes comprised in the liquid medium droplets 43 present on the sample zones 12 can be transferred to a detection substrate 5. As is shown in Fig. 11, fragments (e.g. nanodroplets) 8 of the larger droplets 43 present on the sample zones 12 may be transferred to the detection substrate 5, whereby corresponding spots 9 are formed on the detection substrate 5. The transfer occurs locally per spot. The transfer maintains the orientation of all spots, thereby allowing to correlate the spots 9 on the detection substrate 5 to the sample zones 12 of the sample substrate 11 and hence the immobilized nucleic acid templates 3. The transfer may occur by way of electrohydrodynamics as it is used in the printing devices described herein. It is referred to the above discussion. Remaining liquid 43 present on the sample zones 12 may then be removed and the sample substrate 11 may be washed to remove any remaining released labels (e.g. fluorescent dyes). A new sequencing cycle may then begin. Detection of the transferred detectable labels on the detection substrate may advantageously occur in parallel to further sequencing steps. The fluorescent labels may be detected using an optical detection unit.

Microscope images were taken from fluorescing droplets with a high resolution fluorescene microscope. The droplets contained fluorophores that were cleaved from extended DNA hybrids. Hydrophilic glasses were spotted with the liquid medium containing cleaved fluorophores by way of electrohydrodynamic printing using the printing technology described in EP 2 540 661 A1. Spots with a pitch of 1 to 2 micrometers were spotted at a rate of approx.. 75 microseconds per spot. The smallest individual spots were approx. 1 micrometer in diameter and therefore were well-visible using a high resolution fluorescence microscope.

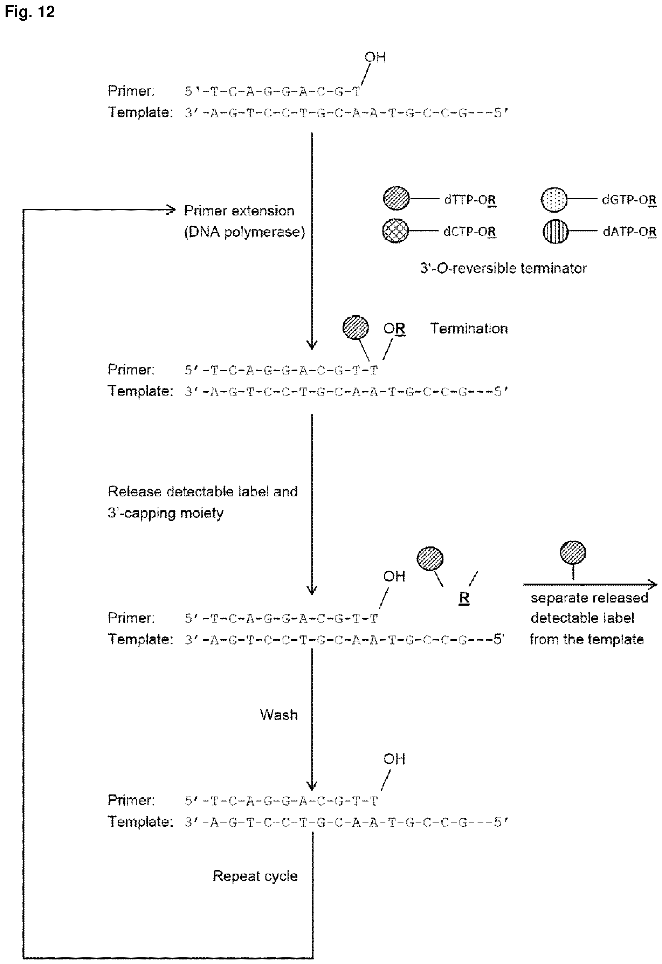

Fig. 12 illustrates core sequencing steps as can be performed using the method of the invention using sequencing by synthesis by cyclic reversible termination. For sequencing, a primer is hybridized to the nucleic acid template, which is immobilized to a surface. Differently labelled 3'-O-reversible terminators are used as hybridizing sequencing reagent comprising a detectable label. In the shown embodiment, each nucleotide analogue comprises a different fluorescent label (one for each base). As discussed herein, also other formats are known and can be used in conjunction with the present invention. The reversible terminating group (shown as R) is provided in the illustrated embodiment as capping moiety which blocks the 3'OH of the pentose of the nucleotide analogues. During primer extension, the DNA polymerase incorporates the matching nucleotide analogue. Further extension is blocked by the capping moiety. Non-incorporated nucleotide analogues are then removed, preferably washed away and the detectable label and the capping moiety R are then released. Depending on the embodiment of nucleotide analogue used, the release of the detectable label and the capping moiety can be performed e.g. (i) in parallel, or (ii) the detectable label can be released prior to the capping moiety or (iii) the capping moiety can be released prior to the detectable label. The detectable label (and optionally the capping moiety) is thereby present in released form in the liquid medium and can then be separated from the immobilized nucleic acid template. As discussed herein, the separated released detectable label can be transferred to a substrate on which it is then subsequently detected. The remaining liquid is then preferably washed away. The free 3'OH group at the extended primer is restored and the next sequencing cycle can begin. As discussed herein, the sequencing reactions can be advantageously performed in a printhead, and the detectable label released in each cycle can be printed on a detection substrate (e.g. as spot), whereby the released detectable label is separated from the immobilized nucleic acid template.

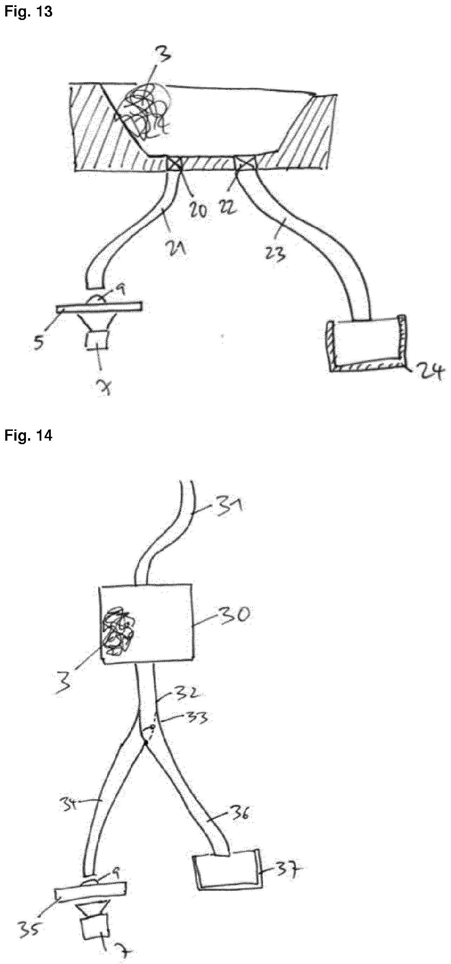

Fig. 13 shows a reservoir with a DNA template 3 in the form of a rolony being arranged in the reservoir. The reservoir has a first valve 20 arranged at the bottom of the reservoir and a first tube 21 attached to the valve 20. By opening the first valve 20, released detectable label comprised in a liquid medium in the reservoir can be transferred from the reservoir through the first tube 21 to a bypass-location on a detection substrate 5 and form a spot 9 on the detection substrate 5. The detection substrate 5 is transparent and arranged above a sensor 7. The sensor 7 can analyse released detectable label present in the spot 9 through the transparent detection substrate 5. Closing the first valve 20 prevents any liquid from flowing into the first tube 21 and hence to the bypass-location. The reservoir has a second valve 22 arranged at the bottom of the reservoir and a second tube 23 attached to the second valve 22. By opening the second valve 22, remainders of the fluid present in the reservoir and/or a wash-fluid in the reservoir can be discharged from the reservoir through the second tube 23 to a drainage-location 24. The reservoir is open at the top hence allowing the introduction of fluids necessary to perform the method steps into the reservoir.

Fig. 14 shows a reservoir in the form of a microfluidic channel 30. A DNA template 3 in the form of rolony is arranged in the microfluidic channel 30. The reservoir has a feed tube 31 and an exit tube 32. A switch or gate 33 is arranged at the end of the exit tube 32. The switch 33 can be switched between the position shown in solid lines in Fig. 4 and a second position indicated by broken lines in Fig. 4. In the second position, the switch 33 connects the exit tube 32 with a first tube 34. This allows a released detectable label present in a liquid medium comprised in the reservoir to be transferred from the reservoir through the first tube 34 to a bypass-location on a substrate 35 and form a spot 9 on a substrate 35. The substrate 35 is transparent and arranged above a sensor 7. The sensor 7 can analyse released detectable label present in the spot 9 through the detection substrate 35. Changing the switch 33 to the first position prevents any liquid from flowing into the first tube 34 and hence to the bypass-location. In the first position, the switch 33 connects the exit tube 32 with a second tube 36. In doing so, remainders of the fluid present in the reservoir and/or a wash-fluid in the reservoir can be discharged from the reservoir through the second tube 36 to a drainage-location 37. The feed tube 31 allows the introduction of fluids necessary to perform the method steps into the reservoir.

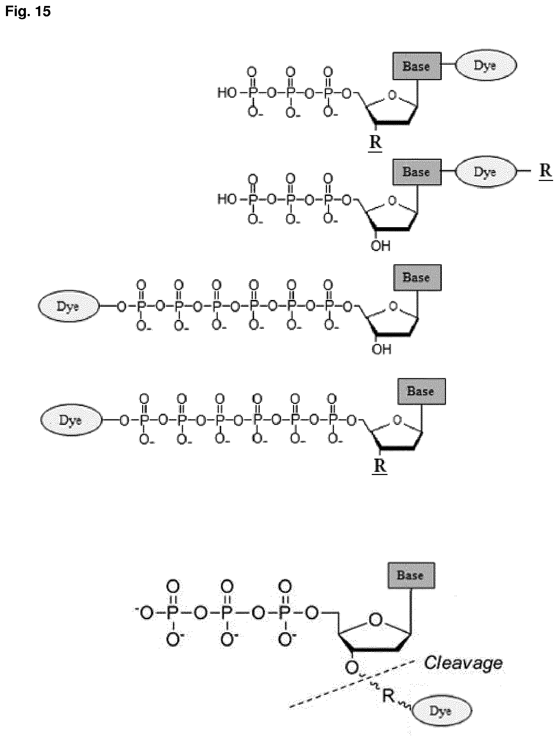

Fig. 15 shows different exemplary embodiments for reversible terminators that can be used for sequencing (modified from Chen 2013, Chen 2014 and Kim 2014). The first shows a 3'O-blocked reversible terminator, wherein the detectable label, here a fluorescent dye, is attached to the base and wherein the 3'OH group is blocked by a reversible terminating group R. The second shows a 3'unblocked reversible terminator, wherein the reversible terminating group R is attached to the fluorescent dye that is attached to the base. The third shows a dye-labelled hexaphosphate nucleotide. The fourth shows a dye-labelled hexaphosphate nucleotide, wherein the 3'OH group is blocked by a reversible terminating group R. The fifth shows an embodiment wherein the fluorescent dye is attached to the reversible terminating group R.

### Cited references

Goodwin et al., Nature Reviews, June 2016, Vol. 17: pp. 333 - 351 "Coming of age: ten years of next-generation sequencing technologies"
Yohe et al., Arch Pathol Lab Med, November 2017, Vol. 141: pp. 1544 - 1557 "Review of Clinical Next-Generation Sequencing"
Masoudi-Nejad, Chapter 2 "Emergence of Next-Generation Sequencing in "Next Generation Sequencing and Sequence Assembly" SpringerBriefs in Systems Biology, 2013
Chen et al, 2013 "The History and Advances of Reversible Terminators Used in New Generations of Sequencing Technology" Genomics Proteomics Bioinformatics 11 (2013) 34-40
Chen et al, 2014, "DNA polymerases drive DNA sequencing-by synthesis technologies: both past and present", Frontiers in Microbiology, Volume 5, Article 305, p. 1 to 11
Kim, 2014 "Synthesis of 3'-O-fluorescently mono-modified reversible terminators and their uses in sequencing-by-synthesis"; Bioorganic & Medicinal Chemistry Letters Volume 24, Issue 1, Pages 209-213
Xu, Mingya, "Nex-Generation Sequencing for Biomedical Applications" (2013), Biomedical Sciences ETDs, Paper 152
US2014/0242579; WO2005/084367; WO2017/139415; WO2017/139419; EP 1 477 230 A1; WO 2013/000558; WO2016/12081; EP 2 540 661 A1

## Claims

1. A method for determining the sequence of a nucleic acid template comprising
a) contacting the nucleic acid template with a hybridizing sequencing reagent comprising a detectable label under suitable conditions to provide a hybrid comprising a detectable label;
b) releasing the detectable label from the hybrid; and
c) separating released detectable label from the nucleic acid template;
wherein the method further comprises d) detecting separated detectable label for determining the sequence.

2. The method according to claim 1, **characterized in that** separating in step c) involves transferring released detectable label to a detection substrate.

3. The method according to claim 1 or 2, wherein the hybridizing sequencing reagent comprising a detectable label is a nucleotide analogue comprising a nucleobase, a detectable label and a reversible terminating group.

4. The method according to one or more of claims 1 to 3, wherein the released detectable label is comprised in a liquid medium and the method comprises printing released detectable label onto a detection substrate.

5. The method according to one or more of claims 1 to 4, having one or more of the following characteristics:
(i) the method comprises
- providing a printing device, wherein the nucleic acid template is comprised in the printing device, and
- performing steps a) and b) in the printing device, and
- wherein step c) comprises printing released detectable label comprised in a liquid medium onto a detection substrate;
(ii) the printing device comprises a reservoir and a nozzle, wherein optionally, the reservoir is provided by the nozzle;
(iii) the method comprises
- providing a printing device comprising a reservoir and a nozzle, wherein the nucleic acid template is comprised in the reservoir and wherein optionally, the reservoir is provided by the nozzle, and
- performing steps a) and b) in the reservoir, and
- wherein step c) comprises transferring released detectable label comprised in a liquid medium through the nozzle onto a detection substrate.

6. The method according to one or more of claims 1 to 5, comprising performing repeated sequencing cycles Xn comprising steps a) to c), wherein the method comprises
- providing a printing device comprising a nozzle, wherein the nucleic acid template is comprised in the printing device, preferably in a reservoir,
- performing a first sequencing cycle X1 comprising
a1) contacting within the printing device the nucleic acid template with a sequencing reagent comprising a detectable label under suitable conditions to provide a hybrid comprising a detectable label;
b1) releasing the detectable label from the hybrid into a liquid medium comprised in the printing device; and
c1) separating released detectable label from the nucleic acid template that is comprised in the printing device wherein separating comprises transferring released detectable label comprised in the liquid medium through the nozzle onto a detection substrate, thereby providing at least one spot comprising the detectable label released during the first sequencing cycle on the detection substrate;
- performing a second sequencing cycle X2 comprising
a2) contacting within the printing device the nucleic acid template obtained after the first sequencing cycle with a sequencing reagent comprising a detectable label under suitable conditions to provide a hybrid comprising a detectable label;
b2) releasing the detectable label from the hybrid into a liquid medium comprised in the printing device; and
c2) separating released detectable label from the nucleic acid template that is comprised in the printing device wherein separating comprises transferring released detectable label comprised in the liquid medium through the nozzle onto a detection substrate, thereby providing at least one spot comprising the detectable label released during the second sequencing cycle on the detection substrate;
- performing further subsequent sequencing cycles Xn comprising steps an) to cn), wherein step cn) of each further sequencing cycle comprises separating released detectable label from the nucleic acid template that is comprised in the printing device wherein separating comprises transferring released detectable label comprised in a liquid medium through the nozzle onto a detection substrate, thereby providing as result of each subsequent sequencing cycle at least one transferred spot comprising the detectable label released during each subsequent sequencing cycle on a detection substrate;
wherein the method further comprises detecting the spotted detectable labels for determining the sequence of the nucleic acid template.

7. The method according to one or more of claims 4 to 6, having one or more of the following characteristics:
(i) released detectable label comprised in a liquid medium is discharged as one or more droplets from the nozzle onto the detection substrate to form a spot in a definite location on the detection substrate;
(ii) the nozzle of the printing device does not contact the detection substrate during the printing process;
(iii) the printing device has a nozzle that is suitable to create a drop out of a meniscus of a liquid arranged in the nozzle, wherein the created drop is urged to travel from the meniscus towards the substrate, if liquid is placed into the nozzle;
(iv) the printing device has an electrode that is suitable to come into contact with a liquid, if the liquid is placed into the nozzle and has a counter-electrode; wherein optionally/preferably the counter-electrode is in contact with the detection substrate;
(v) the printing device allows to discharge nanodroplets out of a liquid continuum by applying an electric field; and/or
(vi) the printing device is an electrohydrodynamic printing device.

8. The method according to one or more of claims 1 to 7, **characterized in that** the method is performed using a printing device comprising a multitude of printing units wherein each printing unit comprises or is connected to a nozzle.

9. The method according to one or more of claims 4 to 8, **characterized in that** the method is performed using a printing device comprising an arrangement or array of printing units, each printing unit comprising or being connected to a nozzle and each printing unit comprising a nucleic acid template to be sequenced, wherein steps a) to c) are performed in parallel in each printing unit comprising a nucleic acid template and wherein step c) comprises transferring released detectable label comprised in a liquid medium through each nozzle onto a detection substrate.

10. The method according to one or more of claims 1 to 3, **characterized in that** the nucleic acid template is immobilized to a sample substrate.

11. The method according to claim 10, comprising
a) contacting the immobilized nucleic acid template with a hybridizing sequencing reagent comprising a detectable label under suitable conditions to provide a hybrid comprising a detectable label;
b) releasing the detectable label from the hybrid into a liquid medium; and
c) separating released detectable label from the nucleic acid template that is immobilized to the sample substrate by transferring released detectable label comprised in the liquid medium from the sample substrate to a location on a separate detection substrate;
wherein the method further comprises detecting the separated detectable label transferred to the detection substrate for determining the sequence.

12. The method according to claim 10 or 11, wherein step c) comprises transferring the released detectable label comprised in the liquid medium in form of droplets from the sample substrate to the definite location on the separate detection substrate, wherein preferably, the transfer to the detection substrate maintains the arrangement or array of all sample zones.

13. The method according to one or more of claims 10 to 12, comprising
- providing a sample substrate comprising different nucleic acid templates immobilized in definite sample zones on the sample substrate thereby providing a sample zone arrangement or array, and
- performing steps a) and b) for different nucleic acid templates immobilized on the sample substrate, and
- wherein for the different nucleic acid templates step c) comprises separating released detectable label from the nucleic acid template that is immobilized to a sample zone by transferring released detectable label comprised in the liquid medium from the sample zone to a definite location on a detection substrate.

14. The method according to one or more of claims 10 to 13, comprising performing repeated sequencing cycles Xn comprising steps a) to c), wherein the method comprises
- immobilizing the nucleic acid template to a sample zone provided on a sample substrate,
- performing a first sequencing cycle X1 comprising
a1) contacting the immobilized nucleic acid template with a hybridizing sequencing reagent comprising a detectable label under suitable conditions to provide a hybrid comprising a detectable label;
b1) releasing the detectable label from the hybrid into a liquid medium applied to the sample zone where the nucleic acid template is immobilized; and
c1) separating released detectable label from the nucleic acid template that is immobilized to the sample zone by transferring released detectable label comprised in the liquid medium from the sample zone to a location on a separate detection substrate, thereby providing at least one spot comprising the detectable label released during the first sequencing cycle on the detection substrate;
- performing a second sequencing cycle X2 comprising
a2) contacting the nucleic acid template obtained after the first sequencing cycle which is immobilized to the sample zone with a hybridizing sequencing reagent comprising a detectable label under suitable conditions to provide a hybrid comprising a detectable label;
b2) releasing the detectable label from the hybrid into a liquid medium applied to the sample zone where the nucleic acid template is immobilized; and
c2) separating released detectable label from the nucleic acid template that is immobilized to the sample zone by transferring released detectable label comprised in the liquid medium from the sample zone to a location on a separate detection substrate, thereby providing at least one spot comprising the detectable label released during the second sequencing cycle on the detection substrate;
- performing further subsequent sequencing cycles Xn comprising steps an) to cn), wherein step cn) of each further sequencing cycle comprises separating released detectable label from the nucleic acid template that is immobilized to the sample zone by transferring released detectable label comprised in the liquid medium from the sample zone to a location on a separate detection substrate, thereby providing as result of each subsequent sequencing cycle at least one spot comprising the detectable label released during the subsequent sequencing cycle on the detection substrate;
wherein the method further comprises detecting the transferred detectable labels for determining the sequence of the nucleic acid template.

15. The method according to one or more of claims 1 to 14, preferably 10 to 14, comprising printing one or more liquid mediums, preferably liquid reaction mediums, onto the sample zone(s) comprising the immobilized nucleic acid template, wherein optionally printing occurs using a printing device comprising multiple nozzles, wherein optionally, the nozzles are arranged as array.

16. A sequencing system for performing the sequencing method according to any one of claims 1 to 15, comprising a printing device.

17. The sequencing system according to claim 16, having one or more of the following characteristics:
(i) the printing device comprises a reservoir and a nozzle, wherein a nucleic acid template can be immobilized in the reservoir;
(ii) a nucleic acid template is immobilized in the printing device, preferably in reservoir comprising or being connected to a nozzle;
(iii) the printing device comprised multiple printing units each comprising a nozzle, wherein optionally, different nucleic acid templates are immobilized in different printing units; and/or
(iv) the printing device comprises a heating and/or cooling unit which allows to adjust the temperature in the reservoir;
(v) it comprises a detection substrate, wherein optionally, the detection substrate can be moved relative to the nozzle;
(vi) it comprises a first printing device, comprising at least one sample zone for immobilizing a nucleic acid template, and optionally comprises a second printing device adapted to supply fluids to the first printing device.

18. Use of a printing device for transferring a detectable label that was released from a nucleic acid hybrid generated in the course of a sequencing reaction to a bypass location, preferably a detection substrate, wherein the transferred detectable label is detected to identify the detectable label, wherein preferably, the printing device comprises a nucleic acid template.

19. The use according to claim 18, wherein the printing device comprises a reservoir and wherein the nucleotide template is immobilized in the reservoir, and wherein preferably, the sequencing reaction is performed in the reservoir and wherein detectable label released during the sequencing reaction is printed through a nozzle onto a discrete location on a detection substrate.
